# EUROPÄISCHE PATENTANMELDUNG

(11) **EP 4 729 939 A2**
(43) Veröffentlichungstag der Anmeldung: **22.04.2026**
(21) Anmeldenummer: 26163352.3
(22) Anmeldetag: 14.06.2024
(51) Int. Cl.: G01N 33/493

(54) **DETEKTIONSVORRICHTUNG ZUR BESTIMMUNG EINES FREIGABEKRITERIUMS FÜR EIN FÜLLSTANDSBESTIMMUNGSSYSTEM**

(30) Priorität: 16.06.2023 DE 102023115879
(62) Teilanmeldung aus: 24735899.7
(71) Anmelder: Elixion Medical GmbH, 40217 Düsseldorf (DE)
(72) Erfinder: Roser, Mario, 80336 München (DE); Fendt, Ludwig, 80801 München (DE)
(74) Vertreter: MFG Patentanwälte Meyer-Wildhagen Meggle-Freund Gerhard PartG mbB

(57) **Zusammenfassung**

Behälter (300) für ein Füllstandsbestimmungssystem (1) zum Bestimmen eines Füllstandskriteriums eines medizinischen Fluides, wobei der Behälter (300) dazu eingerichtet ist, das medizinische Fluid aufzunehmen, der Behälter (300) umfassend eine Halterung, mittels der eine Füllstandsmessanordnung (200) und der Behälter (300) miteinander verbindbar eingerichtet sind, wobei die Halterung an dem Behälter (300) eine Tasche (307, 308) umfasst, die dazu ausgestaltet ist, die Füllstandsmessanordnung (200) nach einem Schwert-Scheide-Prinzip aufzunehmen.

## Beschreibung

### GEBIET DER ERFINDUNG

Die vorliegende Erfindung bezieht sich allgemein und insbesondere auf eine Detektionsvorrichtung zur Bestimmung eines Freigabekriteriums für ein Füllstandsbestimmungssystem. Weiterhin bezieht sich die vorliegende Erfindung auf ein Füllstandsbestimmungssystem, einen Behälter für ein Füllstandbestimmungssystem, und ein Verfahren zur Bestimmung eines Freigabekriteriums für ein Füllstandsbestimmungssystem.

### HINTERGRUND DER ERFINDUNG

Es sind allgemein Behälter zum Aufnehmen medizinischer Fluide bekannt. Diese Behälter können als Urinbeutel oder Urinmesssystem Urin eines Patienten zur Überwachung und Pflege des Patienten aufnehmen. Weiterhin können die Behälter bei Drainagen Fluide des Patienten aufnehmen, die beispielsweise nach Operationen aus Wunden entnommen werden sollen. Zudem können die Behälter medizinische Fluide an einen Patienten abgeben, beispielsweise als Infusionen (Infusionsbeutel). Bei der Verwendung aller dieser Behältertypen, die medizinische Fluide aufnehmen, gehört immer eine Bestimmung der Veränderung des Füllstandes in dem Behälter über den Behandlungsverlauf zur Überwachung und Pflege des Patienten.

Die Überwachung und Pflege des Patienten kann dabei in Krankenhäusern, auf Normalstationen, Intensivstationen, in Pflegeheimen oder der häuslichen Pflege erfolgen.

Weiterhin ist bekannt, dass eine Skala zum Ablesen des Füllstands an den Behältern vorgesehen ist. Auch Vorrichtungen zum Messen des Füllstands sind bekannt. Diese nutzen verschiedene Messmethoden, beispielsweise ein Wiegen oder kapazitive Bestimmung des Füllstands.

Aufgabe der vorliegenden Erfindung ist es, ein verbessertes System zur Füllstandsbestimmung eines medizinischen Fluides in einem Behälter zur Verfügung zu stellen.

Bei einer Füllstandsmessung kann es sein, dass eine Lage, eine Beschleunigung oder eine Komprimierung des Behälters den Füllstand verändern und somit auch einen Messfehler auf eine Bestimmung des Füllstandes erzeugen. Weiterhin sind einige Behälter zum Aufnehmen eines medizinischen Fluides flexibel, beispielsweise Beutel. Da flexible Beutel selbst nicht zum vertikalen Stand fähig sind, ist eine genaue Füllstandsmessung nur möglich, wenn diese zuvor mit einem Halterungssystem in eine definierte, vertikale, in der Regel am Patientenbett, seitlich hängende Position gebracht wurden. Beim Transport des Patienten im Bett wird der Beutel jedoch oft abgenommen und in das Patientenbett gelegt, aufgrund des sich folglich frei verformenden Beutels ist eine zuverlässige Füllstandsmessung nicht mehr möglich.

Starke Beschleunigungen können ebenfalls zu Messfehlern führen. Diese können beispielsweise auftreten, wenn der Patient bereits wieder mobilisiert ist und zum Beispiel mit dem Behälter zur Toilette läuft. Die Beschleunigung des Laufens führt zu Lageveränderungen des Behälters und Wellenbildung im Behälter, was eine zuverlässige Füllstandsmessung unmöglich macht.

Zudem kann ein Eindrücken des Behälters ebenfalls zu einem Messfehler führen. Solche Fehler können beispielsweise auftreten, wenn der Patient den seitlich am Bett hängenden Urinbeutel komprimiert, indem z.B. ein Nachttisch auf Rollen vom Patienten näher an das Bett herangezogen wird, wodurch der Urinbeutel zwischen Bett und Nachttisch eingeklemmt wird.

Um das Problem dieser Messfehler zu lösen, ist eine erfindungsgemäße Detektionsvorrichtung vorgesehen, die eine Füllstandsmessung nur zulässt, also ein Freigabekriterium bestimmt, wenn die obigen Messfehler nicht zu erwarten sind.

Eine solche Detektionsvorrichtung ist bei einer Füllstandsmessung an vielen verschiedenen Behältern in den oben genannten Einsatzbereichen vorteilhaft. Um diese Detektionsvorrichtung an den verschiedenen Behältern einsetzten zu können, ist ein erfindungsgemäßes Füllstandsbestimmungssystem vorgesehen, welches die Detektionsvorrichtung umfasst und modular ist, sodass eine Bestimmung eines Freigabekriteriums einer Füllstandsmessung an vielen verschiedenen Behältertypen möglich ist.

US 2020/209044 A1 offenbart ein Leiterplattengerät umfassend: einen ersten kapazitiven Sensor, der so konfiguriert ist, dass er eine erste Kapazität innerhalb eines abgeschlossenen Volumens mit bekannten Abmessungen misst, wobei sich die erste Kapazität ändert, wenn eine Substanz in das abgeschlossene Volumen eingebracht wird; einen zweiten kapazitiven Sensor mit mehreren Auslösepunkten auf mehreren entsprechenden bekannten Höhen innerhalb des abgeschlossenen Volumens, wobei der zweite kapazitive Sensor so konfiguriert ist, dass er erkennt, wann die in das abgeschlossene Volumen eingebrachte Substanz jeweils die entsprechenden bekannten Höhen erreicht hat; und wobei mindestens einer der folgenden Werte - ein Füllstand der Substanz innerhalb des abgeschlossenen Volumens, ein Volumen der Substanz innerhalb des abgeschlossenen Volumens oder eine Durchflussrate der Substanz in das abgeschlossene Volumen - basierend auf Daten des ersten kapazitiven Sensors und des zweiten kapazitiven Sensors bestimmt wird.

### ZUSAMMENFASSUNG DER ERFINDUNG

Nach einem ersten Aspekt stellt die vorliegende Erfindung ein Füllstandsbestimmungssystem zum Bestimmen eines Füllstandskriteriums eines medizinischen Fluides in einem Behälter nach Anspruch 1 dar.

Nach einem zweiten Aspekt stellt die vorliegende Erfindung einen Behälter für ein Füllstandsbestimmungssystem nach Anspruch 14 dar.

Nach einem dritten Aspekt stellt die vorliegende Erfindung ein Verfahren zur Bestimmung eines Freigabekriteriums für ein Füllstandsbestimmungssystem nach Anspruch 15 dar.

Weitere Aspekte und Merkmale der vorliegenden Erfindung ergeben sich aus den abhängigen Ansprüchen, der beigefügten Figuren und der nachfolgenden Beschreibung bevorzugter Ausführungsformen.

### KURZBESCHREIBUNG DER ZEICHNUNG

Ausführungsformen der Erfindung werden nun beispielhaft und unter Bezugnahme auf die beigefügten Figuren beschrieben. Gezeigt ist in der:
- Fig. 1: ein Füllstandsbestimmungssystem nach einem Ausführungsbeispiel;
- Fig. 2: ein Steuergerät für ein Füllstandsbestimmungssystem nach einem Ausführungsbeispiel;
- Fig. 3a: ein Steuergerät nach einem Ausführungsbeispiel;
- Fig. 3b: ein Steuergerät nach einem Ausführungsbeispiel;
- Fig. 4a: eine Füllstandsmessanordnung nach einem Ausführungsbeispiel;
- Fig. 4b: eine Füllstandsmessanordnung nach einem Ausführungsbeispiel;
- Fig. 5a: eine Füllstandsmessanordnung nach einem Ausführungsbeispiel;
- Fig. 5b: eine Füllstandsmessanordnung nach einem Ausführungsbeispiel;
- Fig. 5c: eine Füllstandsmessanordnung nach einem Ausführungsbeispiel;
- Fig. 5d: eine Füllstandsmessanordnung nach einem Ausführungsbeispiel;
- Fig. 6a: ein Behälter nach einem Ausführungsbeispiel;
- Fig. 6b: ein Behälter nach einem Ausführungsbeispiel;
- Fig. 6c: ein Behälter nach einem Ausführungsbeispiel;
- Fig. 6d: ein Behälter nach einem Ausführungsbeispiel;
- Fig. 6e: ein Behälter nach einem Ausführungsbeispiel;
- Fig. 6f: ein Behälter nach einem Ausführungsbeispiel;
- Fig. 6g: ein Behälter nach einem Ausführungsbeispiel;
- Fig. 6h: ein Behälter nach einem Ausführungsbeispiel;
- Fig. 7: ein Steuergerät und eine Füllstandsmessanordnung nach einem Ausführungsbeispiel;
- Fig. 8: ein Steuergerät, eine Füllstandsmessanordnung und einen Behälter nach einem Ausführungsbeispiel;
- Fig. 9a: ein Steuergerät, eine Füllstandsmessanordnung und einen Behälter nach einem Ausführungsbeispiel;
- Fig. 9b: zeigt ein Steuergerät, eine Füllstandsmessanordnung und einen Behälter nach einem Ausführungsbeispiel;
- Fig. 9c: zeigt einen Querschnitt durch die Tasche des Behälters mit der Füllstandsmessanordnung und der rückenseiteigen Spannvorrichtung nach dem Ausführungsbeispiel aus Fig. 9b;
- Fig. 9d: zeigt einen Querschnitt durch die Tasche des Behälters mit der Füllstandsmessanordnung nach einem Ausführungsbeispiel;
- Fig. 9e: zeigt ein Steuergerät, eine Füllstandsmessanordnung und einen Behälter nach einem Ausführungsbeispiel in einer Seitenansicht;
- Fig. 9f: zeigt ein Steuergerät, eine Füllstandsmessanordnung und einen Behälter nach einem Ausführungsbeispiel in einer Seitenansicht;
- Fig. 9g: zeigt einen Querschnitt durch die Tasche des Behälters mit der Füllstandsmessanordnung nach einem Ausführungsbeispiel;
- Fig. 9h: zeigt einen Querschnitt durch die Tasche des Behälters mit der Füllstandsmessanordnung nach dem Ausführungsbeispiel aus Fig. 9g;
- Fig. 9i: zeigt eine Rückseite des Behälters nach einem Ausführungsbeispiel;
- Fig. 9j: zeigt eine Rückseite des Behälters nach einem Ausführungsbeispiel;
- Fig. 9k: zeigt ein Steuergerät, eine Füllstandsmessanordnung und einen Behälter nach einem Ausführungsbeispiel;
- Fig. 9l: zeigt ein Steuergerät, eine Füllstandsmessanordnung und einen Behälter nach einem Ausführungsbeispiel;
- Fig. 9m: zeigt ein Steuergerät, eine Füllstandsmessanordnung und einen Behälter nach einem Ausführungsbeispiel;
- Fig. 10: ein Steuergerät, eine Füllstandsmessanordnung und einen Behälter nach einem Ausführungsbeispiel;
- Fig. 11: ein Steuergerät und eine Füllstandsmessanordnung nach einem Ausführungsbeispiel;
- Fig. 12: ein Steuergerät, eine Füllstandsmessanordnung und einen Behälter nach einem Ausführungsbeispiel;
- Fig. 13: ein Steuergerät, eine Füllstandsmessanordnung und einen Behälter nach einem Ausführungsbeispiel;
- Fig. 14: ein Steuergerät und eine Füllstandsmessanordnung nach einem Ausführungsbeispiel;
- Fig. 15: ein Steuergerät, eine Füllstandsmessanordnung und einen Behälter nach einem Ausführungsbeispiel;
- Fig. 16: ein Flussdiagramm einer beispielhaften Füllstandsmessung; und
- Fig. 17: ein Ausführungsbeispiel eines Mehrzweckcomputers.

### BESCHREIBUNG VON AUSFÜHRUNGSFORMEN

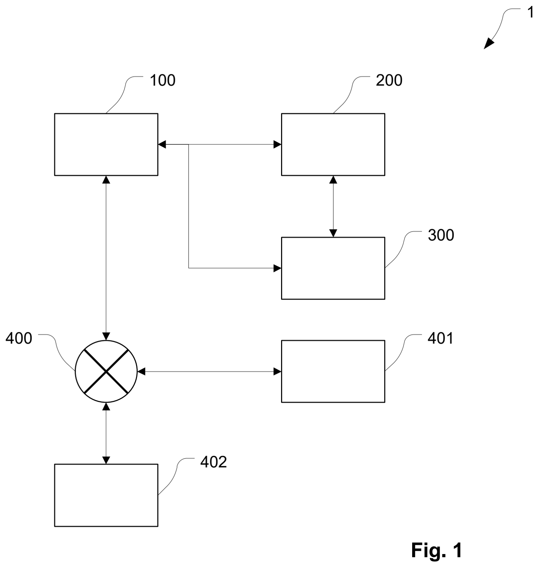

In Fig. 1 ist eine Ausführungsform in Übereinstimmung mit der vorliegenden Erfindung veranschaulicht. Vor einer detaillierten Beschreibung folgen zunächst allgemeine Erläuterungen zu den Ausführungsformen.

Erfindungsgemäß ist eine Detektionsvorrichtung zur Bestimmung eines Freigabekriteriums für ein Füllstandsbestimmungssystem, das einen Füllstand eines medizinischen Fluides in einem Behälter bestimmt, umfassend: einen Lagedetektor, der dazu eingerichtet ist, eine Lage des Füllstandsbestimmungssystems zu bestimmen, einen Beschleunigungsdetektor, der dazu eingerichtet ist, eine Beschleunigung des Füllstandsbestimmungssystems zu bestimmen, wobei Füllstandsmesswerte des Füllstandsbestimmungssystems als Plausibilitätsmessung erfasst werden, wenn die bestimmte Lage innerhalb eines Toleranzbereiches um eine Referenzlage liegt und wenn die Beschleunigung innerhalb eines Toleranzbereiches liegt und das Freigabekriterium anhand der Plausibilitätsmessung erteilt wird, wenn ein Füllstandsmesswert des Füllstandsbestimmungssystems keine überhöhte Änderung zu einem vorherigen Füllstandsmesswert aufweist.

Der Lagedetektor und der Beschleunigungsdetektor können Teil einer Inertial Measurement Unit (IMU) sein. IMUs bestehen aus einem Akzelerometer, das eine Beschleunigung entlang der 3-Achsen (inkl. Erdbeschleunigung) misst und aus einem Gyroskop, das die Winkeländerungsrate (in Grad pro Sekunde) bei Drehung um die 3-Achsen misst.

Die Lagedetektion kann per Akzelerometer durchgeführt werden, beispielsweise entspricht die Beschleunigung in absolut senkrechter Lage entlang der z-Achse 1g und die Beschleunigung auf x- und y-Achse 0g. Dabei ist die absolut senkrechte Lage ein Beispiel einer Referenzlage. Bei einer Neigung projiziert sich die Erdbeschleunigung anteilig auf die x- und y-Achse, wodurch die Lage im Raum berechnet werden kann. So kann einfach überprüft werden, ob sich Behälter und IMU in einer senkrechten Position (innerhalb der Toleranzgrenzen um die Referenzlage) befinden. Dies gilt, solange die relative Ausrichtung zwischen Behälter und IMU konstant ist.

Schräglagen innerhalb des Toleranzbereiches gelten als tolerable Schräglagen, die jedoch unkorrigiert einen Messfehler verursachen würden, diese können jedoch korrigiert werden (wobei die Korrektur von der Geometrie des Behälters abhängt).

Eine Beschleunigungsdetektion kann per Akzelerometer und Gyroskop durchgeführt werden, denn für die 6 Freiheitsgrade der Beschleunigung müssen beide Funktionseinheiten (Akzelerometer und Gyroskop) genutzt werden. Dabei misst das Akzelerometer 3 translatorische Freiheitsgrade und das Gyroskop 3 rotatorische Freiheitsgrade.

Somit kann überprüft werden, ob sich Behälter und IMU in einer ruhenden Position innerhalb der Toleranzgrenzen befinden. Wird hingegen eine zu starke Beschleunigung festgestellt z. B. Patient läuft mit dem System, wird das Freigabekriterium nicht erteilt und damit die Messung verworfen, da die gleiche Beschleunigung auch auf die Flüssigkeit im Behälter wirkt und Wellenbewegungen. eine ungenaue Messung induzieren kann.

Auch anhand der Messung des Füllstands in dem Behälter kann das Freigabekriterium nicht erteilt werden, beispielsweise wenn eine überhöhte Änderung des Füllstandes gemessen wird. Ein überhöhter Anstieg ist dabei beispielsweise eine Füllstandsänderung, die einen vorbestimmten Schwellenwert überschreitet. Dies ist ebenfalls zeitabhängig. Zwischen zwei Füllstandswerten, die eine Stunde auseinander liegen, ist eine größere Änderung plausibel als zwischen zwei Füllstandswerten, die eine Minute auseinander liegen. Somit kann eine überhöhte Änderung gegeben sein, wenn zwischen dem Füllstandsmesswert und dem vorherigem Füllstandsmesswert ein Gradient des Füllstandes über die Zeit überschritten (unterschritten) wird. Alternativ kann für verschiedene zeitliche Abstände zwischen dem Füllstandsmesswert und dem vorherigem Füllstandsmesswert jeweils ein anderer Schwellenwert verwendet sein.

Liegen Beschleunigung und Lage innerhalb der Toleranzen und wird kein überhöhter Anstieg festgestellt, so wird das Freigabekriterium erteilt und die bestimmten Messwerte als plausibel eingestuft. Damit kann aus den plausiblen Messwerten ein Füllstandskriterium bestimmt werden.

Erfindungsgemäß ist ein Füllstandsbestimmungssystem zum Bestimmen eines Füllstandskriteriums eines medizinischen Fluides in einem Behälter, umfassend: den Behälter, der dazu eingerichtet ist, ein medizinisches Fluid aufzunehmen, eine Füllstandsmessanordnung, die dazu eingerichtet ist, Messwerte zu erfassen, die einem Füllstandskriterium des Behälters entsprechen, eine wie oben beschriebe Detektionsvorrichtung, und ein Steuergerät, wobei das Steuergerät und die Füllstandsmessanordnung miteinander verbindbar eingerichtet sind und die Füllstandsmessanordnung und der Behälter miteinander verbindbar eingerichtet sind, wobei das Bestimmen des Füllstandskriteriums in einem verbundenen Zustand des Steuergerätes und der Füllstandsmessanordnung sowie der Füllstandsmessanordnung und des Behälters erfolgt.

Das Steuergerät kann eine Konnektivität aufweisen. Diese kann durch eine integrierte drahtlose Kommunikationstechnologie implementiert sein, wie beispielsweise Bluetooth (auch BLE), WLAN, RFID (auch NFC) oder Mobilfunk, um Messdaten zu übermitteln. Dabei kann eine Onlinefunktion implementiert sein, in der eine Übermittlung an ein Krankenhausinformationssystem (KIS) oder Patientendatenmanagementsystem (PDMS) erfolgt und eine Offlinefunktion, in der eine lokale Zwischenspeicherung erfolgt, sowie eine Übertragung bei der nächsten Verbindung bzw. Onlinefunktion (Push).

Das Füllstandsbestimmungssystem kann den Füllstand in dem Behälter erfassen (inkl. Entleerung) und den Füllstand ausgeben beispielsweise in Millimetern, dazu kann eine Anzeige vorgesehen sein oder der Füllstand per Kommunikationseinheit an einen Server oder ein Endgerät übertragen werden. Die Füllstandsmessung kann beispielsweise eine Genauigkeit von +/- 5% haben.

Das Füllstandsbestimmungssystem integriert in der Detektionsvorrichtung einen Lage- und Beschleunigungssensor, um gültige von ungültigen (z. B. Schräglage, Bewegung, Komprimierung) Messzuständen zu unterscheiden. Damit kann das Freigabekriterium bestimmt werden.

Das Füllstandsbestimmungssystem kann einen oder mehrere Temperatursensoren, welche die Umgebungstemperatur messen, integrieren.

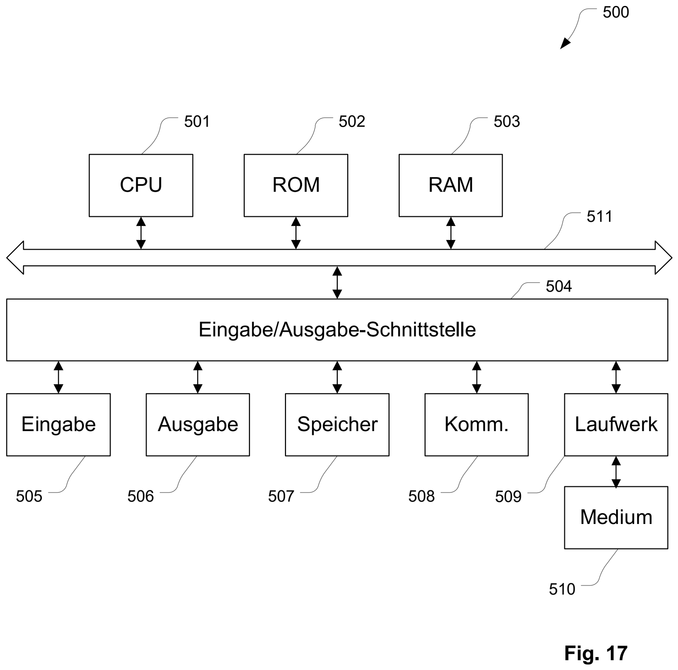

Das Füllstandsbestimmungssystem kann auch Rechenleistung und Speicher (SRAM, Flash, SD-Karte) zur lokalen Datenverarbeitung zur Verfügung stellen. Dies kann vor allem bei einer Implementierung der Offlinefunktion vorgesehen sein. Weiterhin können diese für die Berechnungen der Schritte S12 bis S17 (Fig. 17) genutzt werden.

Das Füllstandsbestimmungssystem kann auch eine Batterie umfassen, zusammen mit (drahtloser) Lade- und Überwachungselektronik, um das System mit Energie zu versorgen. Ein solches Energiemanagement ermöglicht einen Batteriebetrieb. Der Batteriebetrieb kann weiterhin durch ein Laden per Stecker oder ein drahtloses Laden via Induktion vorgesehen sein.

Ein Pairing von zumindest zwei aus dem Steuergerät, dem Behälter und der Füllstandsmessanordnung ermöglicht eine Kompatibilitätsprüfung. Zudem kann durch das Pairing eine Kopplung zu einem Patienten bzw. einer digitalen Patientenakte (auf einem Server) stattfinden. Zu dem Pairing kann eine RFID-Vorrichtung vorgesehen sein, die jeweils in Steuergerät, Behälter und Füllstandsmessanordnung angeordnet ist.

Um eine Beurteilung der Flüssigkeitseigenschaften bei schlechten Lichtverhältnissen zu ermöglichen kann eine Behälterbeleuchtung vorgesehen sein, die den Behälter und das Fluid darin beleuchtet. Diese Beleuchtung kann beispielsweise über eine Detektion einer Annäherung oder einer Geste aktiviert werden, beispielsweise für ein bestimmtes Zeitintervall. Gleichermaßen kann die Anzeige über eine Detektion einer Annäherung oder einer Geste aus einem Standby-Zustand eingeschaltet werden.

Über die Konnektivität können beispielsweise Endgeräte wie ein Smartphone oder eine Smartwatch angebunden werden, beispielsweise mittels einer App. Dadurch können den Pflegekräften die Messwerte auf ein Endgerät übermittelt werden und mit den entsprechenden generierten Visualisierungen angezeigt werden. Die Visualisierungen können beispielsweise auf einem Server oder dem Endgerät generiert sein und einen Füllstandsverlauf über die Zeit umfassen. Dieser Server kann auch das KIS oder PDMS umfassen. Ferner kann den Pflegekräften über Mitteilungen Handlungsbedarf signalisiert werden. Ein Handlungsbedarf ist beispielsweise ein voller Behälter, wobei zusätzliche Patientendaten angegeben werden können, was auch einen bestimmen Raum in dem Krankenhaus oder der Pflegeeinrichtung umfasst.

Auch kann den Pflegekräften ein ungefährer Standort des Füllstandsbestimmungssystems angezeigt werden, beispielsweise ein bestimmtes Gerät in einem bestimmten Gebäude auf einem bestimmten Stockwerk in einem bestimmten Raum.

Eine Konnektivität mittels RFID ermöglicht per Ansteuerung einer RFID-Vorrichtung des Smartphones ein Auslesen von einem RFID-Tag des Behälters, und damit eine Zuordnung, ob der Behälter benutzt oder unbenutzt ist bzw. zu welchem Patienten der Behälter zugeordnet ist oder wie lange sich der Behälter bereits in Verwendung befindet.

Eine Konnektivität mittels RFID ermöglicht per Ansteuerung einer RFID-Vorrichtung des Smartphones die Kommunikation mit dem Steuergerät z. B. für Set-up-Prozesse, in denen die Verbindung zu einem drahtlosen Netzwerk hergestellt wird, Patientendaten übertragen werden oder eine Verbindung zu einer Patientenakte geschaffen wird.

Um eine Modularität zwischen dem Steuergerät und der Füllstandsmessanordnung zu gewährleisten, kann ein Halterungsmechanismus für eine austauschbare Füllstandsmessanordnung implementiert sein. Die Haltevorrichtung stellt dabei auch eine elektrische Verbindung zur Datenübertragung her, beispielsweise über ein standardisiertes Interface. Somit dient der Haltemechanismus als kommunizierende Fassung.

Das Füllstandsbestimmungssystem kann mit einem Server kommunizieren, der Daten der verschiedenen Geräte empfängt, wobei die Geräte jeweils einen Behälter, eine Füllstandsmessanordnung, ein Steuergerät und eine Detektionsvorrichtung umfassen. Der Server kann im lokalen Netzwerk oder der Cloud betrieben werden und Daten können gespeichert, visualisiert sowie analysiert werden und an die Krankenhaus-Informationssysteme oder Smartphone/-watch weitergeleitet und dort gespeichert werden. Der Server kann in dem Füllstandsbestimmungssystem umfasst sein.

Mit den Endgeräten ist es über die RFID-Technologie möglich, per Ansteuerung der RFID-Vorrichtung des Smartphones das Auslesen und Beschreiben von RFID-Tags des Behälters durchzuführen.

Um den Batteriebetrieb zu ermöglichen, kann auch eine Ladeinfrastruktur in dem Füllstandsbestimmungssystem umfasst sein. Dies kann ein Wandregal, einen Ständer etc. zur Aufbewahrung mit integrierter Lademöglichkeit umfassen. Der Transfer der elektrischen Energie beim Ladevorgang kann durch ein induktives Laden via Matten, ein induktives Laden via "Puck" - welcher durch Magnete in die richtige Position gebracht wird - oder einen Kontaktstecker mit magnetischer Positionierung erfolgen.

Es gibt Ausführungen, bei denen das Steuergerät und die Füllstandsmessanordnung mittels eines standardisierten Interfaces miteinander verbindbar eingerichtet sind.

Es gibt Ausführungen, bei denen der Behälter eine Halterung umfasst und die Füllstandsmessanordnung und der Behälter mittels der Halterung miteinander verbindbar eingerichtet sind.

Es gibt Ausführungen, bei denen die Halterung an dem Behälter eine flexible Tasche umfasst, die dazu ausgestaltet ist, die Füllstandsmessanordnung nach einem Schwert-Scheide-Prinzip aufzunehmen.

Die Halterung für Füllstandsmessanordnung am Behälter kann beispielsweise an der Vorderseite oder Rückseite des Beutels angebracht sein, genauer gesagt an jeder Fläche des Behälters, die parallel zur Senkrechten ist. Aus der Anbringung ergibt sich die Referenzlage für die Lagebestimmung.

Die Halterung kann eine flexible, dünnwandige, möglichst knapp geschnittene Folientasche z. B. durch auf die Wand aufgeschweißte oder aufgeklebte flexible Folie sein.

Es gibt Ausführungen, bei denen die Füllstandsmessanordnung eine rückenseitige Spannvorrichtung umfasst, die eine Spannkraft in der flexiblen Tasche erzeugt.

Mittels der Spannkraft kann die Folie der Tasche insbesondere auf der Innenseite zwischen Füllstandsmessanordnung und dem Fluid in dem Behälter glatt gezogen werden. Somit werden Luftblasen vermieden, die die Messung verfälschen können. Durch seine Dielektrizitätskonstante eignet sich Luft als Isolator für kapazitive Messungen, wodurch die Luftblasen die Messung verfälschen. Durch die Spannvorrichtung kann eine Spannkraft sowohl orthogonal als auch parallel zur Einschubrichtung der Tasche erzeugt werden.

Es gibt Ausführungen, bei denen der Behälter ein Gegenlager umfasst das dazu eingerichtet ist die rückenseitige Spannvorrichtung in der Tasche zu fixieren.

Es gibt Ausführungen, bei denen die rückenseitige Spannvorrichtung mit einem Einrastelement ausgestaltet ist und das Einrastelement (207) in dem Gegenlager (309) einrastet, sodass die Spannkraft erzeugt wird.

Durch das Einrastelement kann eine Spannkraft entlang der Einschubrichtung der Tasche erzeugt werden.

Es gibt Ausführungen, bei denen die rückenseitige Spannvorrichtung mit einem halbröhrenähnlichen Profil ausgestaltet ist.

Durch das halbröhrenähnliche Profil ergibt sich ein Luftpolster entlang der gesamten Rückenseite der Füllstandsmessanordnung, wodurch die Füllstandsmessanordnung von dem Außenbereich außerhalb des Behälters abgeschirmt wird. Somit kann die isolierende Wirkung der Luft hier genutzt werden.

Es gibt Ausführungen, bei denen die rückenseitige Spannvorrichtung aufblasbar ist. Dies ermöglicht ebenfalls ein Luftpolster entlang der gesamten Rückenseite der Füllstandsmessanordnung.

Es gibt Ausführungen, bei denen die rückenseitige Spannvorrichtung eine Federvorrichtung umfasst. Dabei kann durch die Federkraft die Spannkraft erzeugt werden. Zudem kann ein Haltemechanismus umfasst sein, der die rückenseitige Spannvorrichtung in einer komprimierten Position der Federvorrichtung hält. Beispielsweise zum Einführen oder Entnehmen der rückenseitigen Spannvorrichtung.

Es gibt Ausführungen, bei denen das Einrastelement als Hebel ausgestaltet ist, der in dem Gegenlager einrastet, wodurch die die rückenseitige Spannvorrichtung von der Füllstandsmessanordnung abgespreizt wird, sodass die Spannkraft erzeugt wird.

Es gibt Ausführungen, bei denen die rückenseitige Spannvorrichtung biegbar ausgestaltet ist und in einem gebogenen Zustand in dem Gegenlager einrastbar ist.

Es gibt Ausführungen, bei denen die Halterung an dem Behälter eine versteifte Tasche umfasst, die dazu ausgestaltet ist, die Füllstandsmessanordnung nach einem Schwert-Scheide-Prinzip aufzunehmen.

Die versteifte Tasche kann eine "Schwertscheide" aus Kunststoff sein, z. B. durch ein auf die Wand aufgeschweißtes oder aufgeklebtes versteiftes Kunststoffteil.

Es gibt Ausführungen, bei denen die versteifte Tasche an einer Wand hin zur Innenseite des Behälters eine Versteifung umfasst.

Es gibt Ausführungen, bei denen die Versteifung ein Rahmen ist.

Es gibt Ausführungen, bei denen die Füllstandsmessanordnung und der Behälter mittels Adhäsion miteinander verbindbar eingerichtet sind.

Eine Befestigung mittels Adhäsion kann beispielsweise per doppelseitigem Klebeband erfolgen, was vorab auf der Füllstandsmessanordnungen angebracht sein kann, welche beispielsweise nach Nutzung mit dem Behälter entsorgt wird.

Es gibt Ausführungen, bei denen die Füllstandsmessanordnung auf den Behälter aufgedruckt ist, wodurch die Füllstandsmessanordnung und der Behälter miteinander verbindbar eingerichtet sind.

Alle Arten der Verbindung von Füllstandsmessanordnung und Behälter müssen den Sensor in zuverlässigen Kontakt mit der Behälterwand bringen, da Luft ein guter dielektrischer Isolator ist und variierende Luftpolster zwischen Sensor und Behälterwand somit zumindest eine kapazitive Messung des Füllstandes des Behälters verfälschen würden. Es kann ein Mechanismus, beispielsweise ein Federblatt, in die rückseitige Innenwand der Tasche oder Schwertscheide integriert sein, welches den Sensor fest an die Behälterwand presst und somit für eine wiederholbare Positionierung sorgt.

Es gibt Ausführungen, bei denen der Behälter einen Urinbeutel umfasst.

Es gibt Ausführungen, bei denen der Behälter ein Urinmesssystem umfasst.

Es gibt Ausführungen, bei denen der Behälter einen Infusionsbeutel umfasst.

Es gibt Ausführungen, bei denen der Behälter eine Thoraxdrainage umfasst.

Es gibt Ausführungen, bei denen der Behälter eine Ventrikeldrainage umfasst.

Es gibt Ausführungen, bei denen der Behälter eine chirurgische Drainage umfasst.

Weitere Behälter finden beispielsweise bei Drainagen in Beutelform, Drainagen in Flaschenform, Infusionsflaschen und Magensonden Anwendung.

Allgemein kann der Behälter ein Ablassventil umfassen, z.B. Quetschventil, welches durch eine elektromechanische Einheit beispielsweise an der Füllstandsmessanordnung betätigt werden kann.

Es gibt Ausführungen, bei denen der Behälter eine RFID-Vorrichtung umfasst.

Die RFID-Vorrichtung kann eine Kopplung des Behälters mit Patienten, der Füllstandsmessanordnung und dem Steuergerät ermöglichen. Durch diese Kopplung können auch Behälter ausgeschlossen werden, beispielsweise aus Kompatibilitätsgründen.

Die RFID-Vorrichtung kann eine Präsenzdetektion des Behälters durch das Steuergerät oder die Füllstandsmessanordnung ermöglichen. Ferner kann eine Identifikation des Behälters ermöglicht sein, sowie ein Schreiben von Patientendaten auf die RFID-Vorrichtung des Behälters, ein Abgleich der Kompatibilität des Behälter und der Füllstandsmessanordnung, ein Abgleich des Behälters und des zugehörigen Patienten, ein Ausschluss von Verwechselungen zwischen Patienten, ein Ausschluss erneuter Verwendung gebrauchter Behälter, ein Ausschluss der Verwendung nicht autorisierter Verbrauchsmaterialien (nicht original, Haltbarkeit abgelaufen, Rückruf etc.).

Die Kopplung von Patient mit Behälter kann bei einer Detektion der RFID-Vorrichtung des Behälters automatisch auslöst werden. Zudem kann bei Koppelung des Steuergerätes mit der RFID-Vorrichtung des Behälters die Patienteninformationen (z. B. Name, Geburtsdatum) automatisch übertragen und auf die RFID-Vorrichtung des Behälters geschrieben werden.

Es gibt Ausführungen, bei denen das Steuergerät dazu eingerichtet ist, mittels einer weiteren RFID-Vorrichtung die RFID-Vorrichtung des Behälters auszulesen und festzustellen, ob der Behälter mit der Füllstandsmessanordnung und dem Steuergerät kompatibel ist und noch nicht verwendet wurde, sodass wenn der Behälter kompatibel ist und noch nicht verwendet wurde, mittels der RFID-Vorrichtung auf die RFID-Vorrichtung des Behälters geschrieben wird, dass der Behälter (nun) verwendet ist.

Es gibt Ausführungen, bei denen beim Schreiben mittels der weiteren RFID-Vorrichtung auf die RFID-Vorrichtung des Behälters ebenfalls patientenbezogene Daten auf die RFID-Vorrichtung des Behälters geschrieben werden.

Es gibt Ausführungen, bei denen das Steuergerät die weitere RFID-Vorrichtung umfasst.

Somit integriert das Steuergerät eine RFID-Vorrichtung, um Behälter zu identifizieren und um mit Endgeräten wie Smartphones bidirektional kommunizieren zu können.

Es gibt Ausführungen, bei denen der Behälter ein Auslassventil umfasst und die Füllstandsmessanordnung einen Aktuator, der dazu eingerichtet ist, das Auslassventil zu betätigen. Der Aktuator kann auch separat von der Füllstandsmessanordnung angeordnet sein und mit dem Steuergerät kommunizierend verbunden sein, um von diesem angesteuert zu werden.

Der Aktuator kann durch das Steuergerät oder eine Elektronikkomponente der Füllstandsmessvorrichtung angesteuert sein, beispielsweise, wenn der Füllstand im Behälter einen vorgegebenen Wert erreicht, beispielsweise ein Maximum. Somit kann ein (elektromagnetisches) Ventil zur automatischen Behälterentleerung angesteuert sein.

Es gibt Ausführungen, bei denen das Steuergerät eine Diagnosevorrichtung umfasst, die dazu eingerichtet ist, das Fluid spektrometrisch zu analysieren.

Die spektrometrische Analyse kann beispielsweise durch ein Minispektrometer und eine breitbandige Strahlungsquelle vorgesehen sein. Dies ist beispielsweise in WO2023/062224 weitergehend erklärt.

Es gibt Ausführungen, bei denen das Steuergerät eine Anzeige umfasst, die dazu eingerichtet ist, Informationen über das bestimmte Füllstandskriterium anzuzeigen.

Die Anzeige kann ein Human-Machine-Interface (HMI) aus Touchscreen und Status-LED implementieren und zur Visualisierung von Information und zur lokalen Bedienung vorgesehen sein.

Es kann ein Helligkeitssensor integriert sein, um die Anzeigehelligkeit der Umgebungshelligkeit anzupassen.

Die Status-LED kann einen Status des Füllstandsbestimmungssystems oder des Patienten - durch Auswertung der Füllstandsmessung - signalisieren, wenn die Anzeige beispielsweise im Stand-by-Zustand ist. Dafür kann die Status-LED verschiedene Farbcodes (grün, gelb, rot) oder Blinkcodes verwenden.

Die Anzeige kann als Touchscreen mit Handschuhen bedienbar ausgelegt sein.

Es gibt Ausführungen, bei denen das Steuergerät einen Präsenzdetektor umfasst, der dazu eingerichtet ist, ein Annähern einer Person oder eine Geste einer Person zu detektieren, wobei aufgrund der Annäherung oder der Geste die Anzeige aus einem Stand-by-Zustand eingeschaltet wird.

Die Person kann in diesem Fall ein Nutzer, z.B. das Pflegepersonal, sein. Eine Geste kann beispielsweise ein Wischen mit der Hand beispielsweise oberhalb der Anzeige oder ein Wischen mit dem Fuß unter dem Füllstandsbestimmungssystem sein (wenn diese seitlich am Patientenbett angebracht ist).

Es gibt Ausführungen, bei denen das Steuergerät eine Energieversorgungsvorrichtung umfasst, die dazu eingerichtet ist, das Steuergerät mit Energie zu versorgen.

Es gibt Ausführungen, bei denen die Füllstandsmessanordnung Elektroden umfasst, die dazu eingerichtet sind, kapazitiv Füllstandsmesswerte zu bestimmen, mittels derer das Füllstandskriterium des Behälters bestimmbar ist.

Dabei können die Messwerte der Elektroden durch Umweltreferenzelektroden, Fluidreferenzelektroden und Temperatursensoren, welche die Umwelt- als auch die Fluidtemperatur erfassen, korrigiert werden.

Zum Anschluss der Füllstandsmessanordnung an das Steuergerät kann ein standardisiertes Interface vorgesehen sein, z.B. I2C, SPI, UART oder CAN-Bus. Ebenfalls kann eine zusätzliche Elektronickomponente an der Füllstandsmessanordnung vorgesehen sein, die ausgebildet ist, um die Sensorik und einen Aktuator anzusteuern. Weiterhin kann ein Befestigungsmechanismus zum austauschbaren Anschluss an das Steuergerät vorgesehen sein, somit ergibt sich eine mechanische Verbindung, während das Interface die Kommunikationsverbindung bereitstellt. Dieser Befestigungsmechanismus ist beispielsweise eine Steckverbindung und ermöglicht ein schnelles Zusammensetzten des (modularen) Füllstandsbestimmungssystems.

Die Dimensionierung der Füllstandsmessanordnung kann mit verschiedenen Ausführungen an verschiedene Behältergrößen angepasst werden. Auch kann das Elektrodendesign, wie oben beschrieben, oder das Trägermaterial je nach Anwendung variiert sein. Ebenfalls kann die Füllstandsmessanordnung mit oder ohne Aktuator, der ein Ablassventil öffnet und schließt, vorgesehen sein. Es kann Varianten geben, bei denen die Füllstandsmessanordnung zumindest aber die Sensoren wie beispielsweise die Elektroden, als Mehrwegteil oder Einwegteil ausgelegt sind, wobei das Einwegteil nach Aufkleben und Nutzung mit dem Behälter entsorgt werden kann.

Die Füllstandsmessanordnung kann einen oder mehrere Temperatursensoren zur Messung der Flüssigkeitstemperatur durch die Beutelwand enthalten.

Es gibt Ausführungen, bei denen die Füllstandsmessanordnung ein Printed Circuit Board umfasst und die Elektroden auf dem Printed Circuit Board ausgebildet sind.

Es gibt Ausführungen, bei denen das Printed Circuit Board formstabil ausgeführt ist.

Es gibt Ausführungen, bei denen das Printed Circuit Board flexibel ausgeführt ist.

Das Trägermaterial der Füllstandsmessanordnung kann ein Rigid PCB, ein Flex PCB (ermöglicht Anpassung an flexible oder runde Behältergeometrien) oder gedruckte Elektronik (auch auf dem Behälter direkt aufgedruckt; ermöglicht kostengünstige Einwegsensoren) sein.

Es gibt Ausführungen, bei denen die Füllstandsmessanordnung eine Elektronikvorrichtung umfasst, die dazu eingerichtet ist, die Bestimmung der Messwerte anhand der Elektroden durchzuführen.

Die Sensorik der Füllstandsmessanordnung umfasst beispielsweise bei einer kapazitiven Messung kontinuierliche Elektroden (Texas Instruments, Ti FDC1004), die in der Referenzlage senkrecht entlang des Behälters verlaufen. Zusätzlich können Umweltreferenzelektroden und Flüssigkeitsreferenzelektroden umfasst sein. Die Elektroden können auch zur senkrechten Richtung schräg verlaufend ausgeführt sein.

Weiterhin können anstatt der kontinuierlichen Elektroden segmentierte Elektroden mit variablen Stufen (Infineon PSoC Cap Sense) vorgesehen sein.

Die Elektroden können auch als parallele Elektroden zur Messung mehrerer Flüssigkeitssäulen nebeneinander vorgesehen sein. Ersatzweise und in Kombination kann eine alternative Methode zur Füllstandsmessung vorgesehen sein. Beispielsweise kann der Füllstand mittels einer induktiven Detektion eines magnetischen Schwimmers in einem Steigrohr oder einer vertikalen Zeile aus Detektoren (segmentierten Präsenzdetektoren z. B. optischen Detektoren (Diodenarray, CCD-Zeile) oder auch ein Array aus Temperatursensoren) oder einer Messung des Beutelgewichts erfolgen. Weitere alternative Methoden können Standardverfahren mit z. B. einem Drucksensor, einem Schwimmer, einem Ultraschallsensor, einem Time of Flight Sensor oder einem Laser sein.

Es kann ein Aktuator umfasst sein, der dazu eingerichtet ist, mittels elektromechanischer Ansteuerung einen Ablass (Ablassventil) zu einer Behälterentleerung im Urinmesssystem-Design durchzuführen. Dazu öffnet der Aktuator ein Ventil des Behälters, beispielsweise ein Quetschventil, das der Aktuator im geschlossenen Zustand einquetschen kann und im geöffneten Zustand nicht einquetscht. Der Aktuator kann auch eine Pumpe umfassen, die beispielsweise ein definiertes Fluidvolumen pro Zyklus pumpt.

Es gibt Ausführungen, bei denen das Steuergerät dazu eingerichtet ist, wenn das Freigabekriterium erteilt ist, anhand der bestimmten Lage des Lagedetektors der Detektionsvorrichtung eine Korrektur des Füllstandskriteriums durchzuführen.

Somit ist das Steuergerät dazu ausgelegt, eine Korrektur von lageabhängigen Messfehlern bei schräg hängendem Behälter durchzuführen.

Erfindungsgemäß ist ein Behälter für ein Füllstandsbestimmungssystem nach einer der oben beschriebenen Ausführungen.

Erfindungsgemäß ist ein Verfahren zur Bestimmung eines Freigabekriteriums für ein Füllstandsbestimmungssystem, das einen Füllstand eines medizinischen Fluides in einem Behälter bestimmt, umfassend der Schritte: Bestimmen einer Lage des Füllstandsbestimmungssystems, Bestimmen einer Beschleunigung des Füllstandsbestimmungssystems, Erfassen von Füllstandsmesswerten des Füllstandsbestimmungssystems als Plausibilitätsmessung, wenn die bestimmte Lage innerhalb eines Toleranzbereiches um eine Referenzlage liegt und wenn die Beschleunigung innerhalb eines Toleranzbereiches liegt, und Erteilen des Freigabekriteriums anhand der Plausibilitätsmessung, wenn ein Füllstandsmesswert des Füllstandsbestimmungssystems keine überhöhte Änderung zu einem vorherigen Füllstandsmesswert aufweist.

Zurückkommend zu Fig. 1 veranschaulicht diese ein Füllstandsbestimmungssystem nach einem Ausführungsbeispiel.

Das Füllstandsbestimmungssystem 1 umfasst ein Steuergerät 100, eine Füllstandsmessanordnung 200 und einen Behälter 300. Weiterhin kann in dem Füllstandsbestimmungssystem 1 ein Netzwerk 400, ein Server 401 und ein Endgerät 402 umfasst sein. Das Füllstandsbestimmungssystem 1 ist modular ausgestaltet, sodass verschiedene Steuergeräte 100, Füllstandsmessanordnungen 200 und Behälter 300 miteinander verbunden werden können oder jeweils gegeneinander ausgetauscht werden können.

Das Steuergerät 100 ist mit der Füllstandsmessanordnung 200 verbunden und empfängt Daten, die für einen Füllstand in dem Behälter 300 indikativ sind. Dies können beispielsweise Messwerte der Füllstandsmessanordnung 200 sein, die direkt von der Sensorik ausgelesen werden, ein gemessener Füllstand oder ein gemessener und um die Lage korrigierter Füllstand. Weiterhin kann das Steuergerät 100 Informationen von der Füllstandsmessanordnung 200 empfangen, die die Ausgestaltung der Füllstandsmessanordnung 200 identifizieren.

Das Steuergerät 100 und alternativ oder zusätzlich auch die Füllstandsmessanordnung 200 sind mit dem Behälter 300 verbunden. Hierbei werden Informationen zu der Ausgestaltung des Behälters 300 übermittelt und ob der Behälter bereits verwendet wurde. Zusätzlich können das Steuergerät 100 und die Füllstandsmessanordnung 200 patientenspezifische Informationen an den Behälter 300 übermitteln und auf einen Speicher, der an dem Behälter 300 umfasst sein kann, schreiben (mittels RFID).

Das Steuergerät 100 kann über das Netzwerk 400 mit dem Server 401 und dem Endgerät 402 kommunizieren. Der Server 401 und das Endgerät 402 können ebenfalls über das Netzwerk miteinander kommunizieren.

Somit kann das Steuergerät 100 Füllstandsmesswerte (das Füllstandskriterium) sowie Informationen, die das Steuergerät 100 oder den Patienten identifizieren, beispielsweise eine Identifikationsnummer (ID) des Gerätes oder Patienten, an den Server 401 übermitteln und der Server 401 kann diese Füllstandsmesswerte in der entsprechenden Patientenakte speichern. Ferner kann das Steuergerät 100 Füllstandsmesswerte (das Füllstandskriterium) sowie Informationen, die das Steuergerät 100 oder den Patienten identifizieren (ID) an das Endgerät 402 übermitteln, wobei das Endgerät 402 dem zuständigen Pflegepersonal zugeordnet ist. Das zuständige Pflegepersonal kann mittels der Patientenakte (und eines Schichtplans) ermittelt sein.

Sowohl Server 401 als auch Endgerät 402 können dazu eingerichtet sein, die Füllstandsmesswerte grafisch darzustellen. Es kann entweder durch das Steuergerät 100, den Server 401 oder das Endgerät 402 ein Handlungsbedarf festgestellt werden, der über das Endgerät 402 dem zuständigen Pflegepersonal mitgeteilt werden kann. Ein Handlungsbedarf kann beispielsweise ein (nahezu) voller Behälter 300 sein.

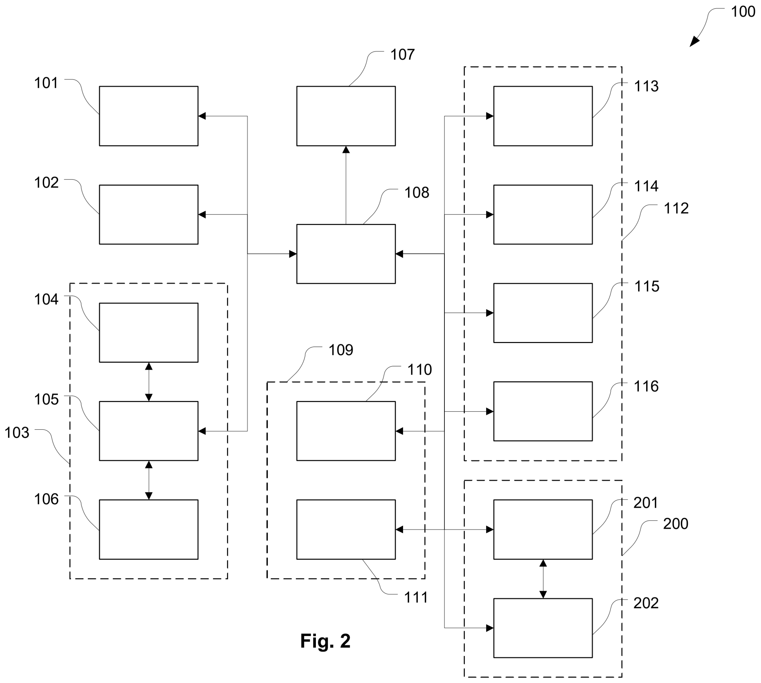

Fig. 2 zeigt ein Steuergerät für ein Füllstandsbestimmungssystem nach einem Ausführungsbeispiel.

Das Steuergerät 100 umfasst einen Mikrocontroller 108, eine Anzeige 107, eine Kommunikationseinheit 109, eine Status-LED 101, eine Behälterbeleuchtung 102, eine Energiemanagementanordnung 103 und eine Sensorikanordnung 112. Ferner ist ebenfalls die wechselbare Füllstandsmessanordnung 200 dargestellt.

Der Mikrocontroller 108 ist dazu ausgestaltet, die Steuerungsfunktionen des Steuergerätes 100 durchzuführen. Weiterhin kann der Mikrocontroller 108 Informationen auf der Anzeige 107 darstellen. Diese Informationen können unter anderem Füllstandsmesswerte, Patientendaten oder Statusinformationen des Steuergerätes wie beispielsweise der Ladezustand sein. Der Mikrocontroller 108 kann ebenfalls einen Speicher aufweisen, auf dem lokal Daten gespeichert werden können. Der Speicher kann auch in dem Steuergerät 100 umfasst sein.

Die Anzeige 107 kann die Informationen darstellen und ebenfalls eine Toucheinheit als Eingabegerät des Steuergerätes 100 umfassen, mit dem ein Nutzer (beispielsweise Pflegepersonal) das Steuergerät 100 bedienen kann.

Ein Status des Patienten, der aufgrund der Füllstandsmesswerte ermittelt ist, kann ebenfalls durch die Status-LED 101 angezeigt sein. Ferner kann die Status-LED auch einen Status des Steuergerätes 100 oder des Füllstandsbestimmungssystems 1 anzeigen. Ein solcher Status kann beispielsweise sein, dass Lage oder Beschleunigung außerhalb des Toleranzbereiches liegen oder eine überhöhte Änderung des Füllstandes erfasst wurde, sodass keine Füllstandsmessung erfolgt (kein Freigabekriterium). Ferner kann ebenfalls eine fehlende Verbindung zu einem Netzwerk, ein voller Behälter oder ein niedriger Ladungszustand angezeigt sein.

Die Behälterbeleuchtung 102 kann eingeschaltet sein, um bei schlechten Lichtverhältnissen (dunkler Raum) das Fluid im Behälter zu überprüfen, beispielsweise anhand der Farbe.

Die Energiemanagementanordnung 103 umfasst eine Ladeeinrichtung 104, einen Ladekontroller 105 und eine Batterie 106. Der Ladekontroller 105 steuert die Energieaufnahme durch die Ladeeinrichtung 104 und die Energiespeicherung und -abgabe durch die Batterie 106, um das Steuergerät 100 (und die Füllstandsmessanordnung 200) mit Energie zu versorgen.

Die Ladeeinrichtung 104 kann eine induktive Ladeeinrichtung sein, die kontaktlos Energie von außerhalb des Steuergerätes 100 aufnimmt. Die Ladeeinrichtung 104 kann auch Energie über einen Stecker aufnehmen.

Die Kommunikationseinheit 109 umfasst Kommunikationsvorrichtungen nach den gängigen Standards, beispielsweise für WLAN 110 oder Mobilfunk 111. Auch eine Kommunikationsvorrichtung für (Low Energy) Bluetooth kann vorgesehen sein. Dadurch kann das Steuergerät 100 mit Geräten wie Servern und Endgeräten direkt oder über ein Netzwerk kommunizieren.

Die Sensorikanordnung 112 umfasst eine inertiale Messeinheit 113 (Inertial Measurement Unit, IMU), einen NFC-Reader 114, einen Präsenzdetektor 115 und eine Diagnosevorrichtung 116.

Die inertiale Messeinheit 113 misst die Beschleunigung und Lage des Steuergerätes 100 und der damit verbundenen Füllstandsmessanordnung 200 sowie dem Behälter (300 in Fig. 1). Somit bestimmt die inertiale Messeinheit 113 die Beschleunigung und Lage für die Feststellung des Freigabekriteriums der Füllstandsmessung durch die Füllstandsmessanordnung 200.

Der NFC-Reader 114 ist eine RFID-Vorrichtung, die mit einer RFID-Vorrichtung im Behälter (300 in Fig. 1) oder einem Endgerät (402 in Fig. 1) kommunizieren kann. Bei der Kommunikation können beispielsweise Informationen zur Identifikation des Steuergerätes 100, der Füllstandsmessanordnung, des Behälters und des Endgerätes übermittelt werden, um beispielsweise eine Kompatibilität zueinander zu überprüfen. Ebenfalls können bei der Kommunikation Patientendaten und ein Benutzungszustand des Behälters übertragen werden.

Der Präsenzdetektor 115 detektiert eine Präsenz oder eine Geste eines Nutzers (einer Pflegekraft). Aufgrund der Detektion kann dann die Anzeige 107 aus einem Stand-by-Zustand angeschaltet werden oder die Behälterbeleuchtung 102 eingeschaltet werden. Der Präsenzdetektor 115 kann einen Bewegungsmelder oder eine Kamera (infrarot) umfassen.

Die Diagnosevorrichtung 116 umfasst eine breitbandige Strahlungsquelle und einen optischen Detektor, beispielsweise ein Spektrometer zur spektrometrischen Analyse des medizinischen Fluides, das durch einen Schlauch aus dem oder in den Behälter 300 fließt.

Ferner kann in der Sensorikanordnung 112 auch ein Helligkeitsdetektor angeordnet sein, der zum Detektieren der Helligkeit in der Umgebung des Steuergerätes 100 eingerichtet ist. Auf Basis der Helligkeit kann dann die Helligkeit der Anzeige 107 eingestellt werden.

Der Stand-by-Zustand der Anzeige kann aufgrund eines vergangenen Zeitintervalls und beispielsweise der detektierten Helligkeit eingeleitet werden.

Die Füllstandsmessanordnung 200 umfasst eine Elektronikeinheit (Elektronikkomponente) 201 mit Elektroden zur Füllstandsmessung und einen Aktuator 202, der in manchen Ausführungen angeordnet sein kann. Der Aktuator 202 bedient ein Auslassventil an dem Behälter (300 in Fig. 1).

Der Aktuator 202 kann durch den Mikrocontroller 108 oder die Elektronikeinheit 201 angesteuert werden, beispielsweise, wenn der gemessene Füllstand ein gewisses Niveau erreicht hat und gerade kein Zufluss detektiert wird.

Die Sensorikanordnung 112 oder Teile der Sensorikanordnung 112 können auch an der Füllstandsmessanordnung 200 angeordnet sein, wobei Auswertungsschritte in der Feststellung des Freigabekriteriums ebenfalls von der Elektronikeinheit 201 durchgeführt werden können.

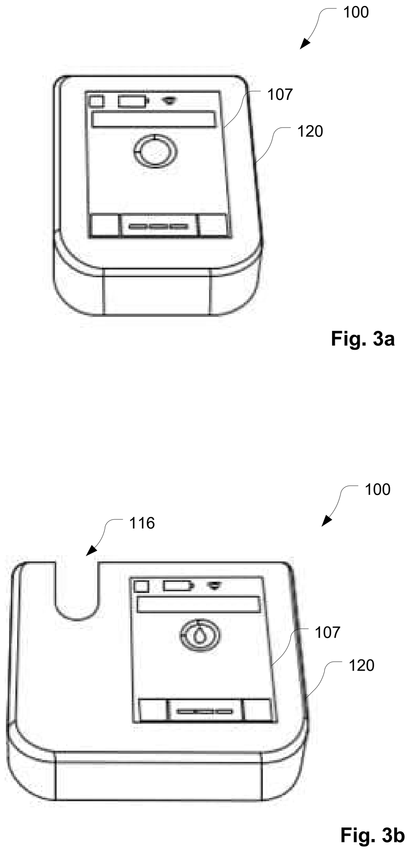

Fig. 3a zeigt ein Steuergerät nach einem Ausführungsbeispiel.

Das Steuergerät 100 umfasst eine Anzeige 107 an der Oberseite eines Gehäuses 120, welches integrierte Schaltkreise und Anordnungen umfasst, die die in Fig. 2 dargestellten Funktionsblöcke implementieren.

Fig. 3b zeigt ein Steuergerät nach einem Ausführungsbeispiel.

Das Steuergerät 100 umfasst eine Anzeige 107 an der Oberseite eines Gehäuses 120, welches integrierte Schaltkreise und Anordnungen umfasst, die die in Fig. 2 dargestellten Funktionsblöcke implementieren.

Weiterhin umfasst das Steuergerät 100 die Diagnosevorrichtung 116 in einem Gehäuseabschnitt, der einen Schlauch, der in den Behälter (300 in Fig. 1) führt, aufnehmen kann.

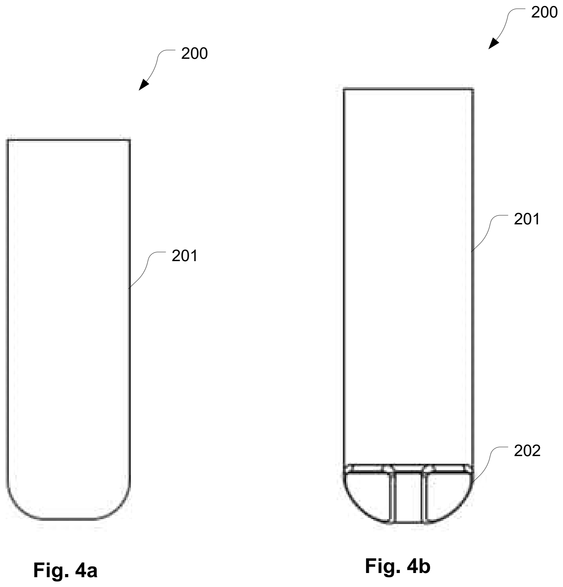

Fig. 4a zeigt eine Füllstandsmessanordnung nach einem Ausführungsbeispiel.

Die Füllstandsmessanordnung 200 ist als Messschwert ausgestaltet und umfasst in dem Messschwert einen Abschnitt, der die Elektronikeinheit 201 mit Elektroden zur Füllstandsmessung des Füllstandes im Behälter aufnimmt.

Fig. 4b zeigt eine Füllstandsmessanordnung nach einem Ausführungsbeispiel.

Die Füllstandsmessanordnung 200 ist als Messschwert ausgestaltet und umfasst in dem Messschwert einen Abschnitt, der die Elektronikeinheit 201 mit Elektroden zur Füllstandsmessung des Füllstandes im Behälter aufnimmt, sowie einen zweiten Abschnitt, der als Halterung fungieren kann und den Aktuator 202 aufnimmt.

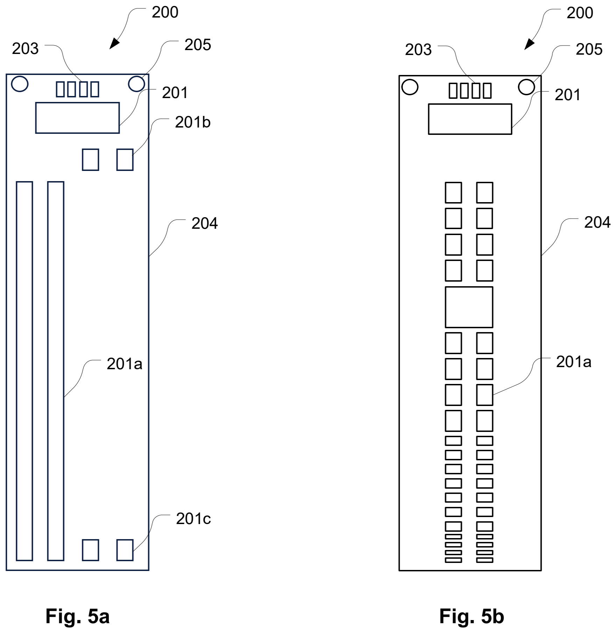

Fig. 5a zeigt eine Füllstandsmessanordnung nach einem Ausführungsbeispiel.

Die Füllstandsmessanordnung 200 umfasst eine Elektronikeinheit 201, Messelektroden 201a, Umgebungsreferenzelektroden 201b, Fluidreferenzelektroden 201c und ein standardisiertes Interface 203 auf einem Trägermaterial 204 und einen Befestigungsmechanismus 205.

Das Trägermaterial 205 kann ein flexibles, steifes oder gedrucktes Printed Circuit Board sein. In dem Trägermaterial 205 sind die elektrischen Verbindungen der Komponenten, die auf dem Trägermaterial 205 angeordnet sind, ausgestaltet.

Durch die Elektronikeinheit 201 wird eine Kapazität jeweils zwischen den Messelektroden 201a, den Umgebungsreferenzelektroden 201b und den Fluidreferenzelektroden 201c bestimmt. Die Kapazität zwischen den Messelektroden 201a wird durch die gemessenen Werte der Referenzelektroden korrigiert.

Die korrigierten Kapazitätswerte oder ein daraus von der Elektronikeinheit 201 bestimmter Füllstand können von der Elektronikeinheit 201 über das standardisierte Interface 203 an das Steuergerät (100 in Fig. 1 und 2) abgegeben werden.

Die Füllstandsmessanordnung 200 ist weiterhin durch den Befestigungsmechanismus 205 mit dem Steuergerät verbindbar ausgestaltet. Der Befestigungsmechanismus 205 umfasst beispielsweise Pins, die in das Steuergerät gesteckt werden können.

Fig. 5b zeigt eine Füllstandsmessanordnung nach einem Ausführungsbeispiel.

Die Füllstandsmessanordnung 200 umfasst eine Elektronikeinheit 201, segmentierte Messelektroden 201a und ein standardisiertes Interface 203 auf einem Trägermaterial 204 und einen Befestigungsmechanismus 205.

Das Trägermaterial 205 kann ein flexibles, steifes oder gedrucktes Printed Circuit Board sein. In dem Trägermaterial 205 sind die elektrischen Verbindungen der Komponenten, die auf dem Trägermaterial 205 angeordnet sind, ausgestaltet.

Durch die Elektronikeinheit 201 wird eine Kapazität jeweils zwischen den segmentierten Messelektroden 201a bestimmt. Der Füllstand kann aus einem Vergleich der Kapazitäten zwischen den einzelnen Segmenten, die auf gleicher Höhe angeordnet sind, bestimmt werden.

Der Füllstand kann von der Elektronikeinheit 201 über das standardisierte Interface 203 an das Steuergerät (100 in Fig. 1 und 2) abgegeben werden.

Die Füllstandsmessanordnung 200 ist weiterhin durch den Befestigungsmechanismus 205 mit dem Steuergerät verbindbar ausgestaltet. Der Befestigungsmechanismus 205 umfasst beispielsweise Pins, die in das Steuergerät gesteckt werden können.

Nicht dargestellt aber ebenfalls enthalten sein können die in Fig. 5a gezeigten Referenzelektroden.

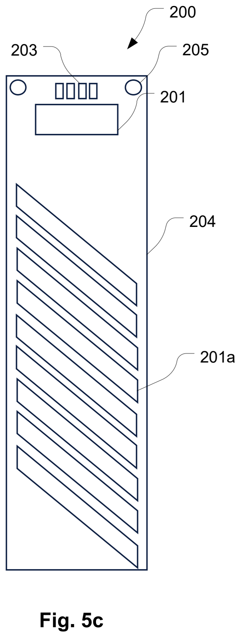

Fig. 5c zeigt eine Füllstandsmessanordnung nach einem Ausführungsbeispiel.

Die Füllstandsmessanordnung 200 umfasst eine Elektronikeinheit 201, segmentierte Messelektroden 201a und ein standardisiertes Interface 203 auf einem Trägermaterial 204 und einen Befestigungsmechanismus 205.

Das Trägermaterial 205 kann ein flexibles, steifes oder gedrucktes Printed Circuit Board sein. In dem Trägermaterial 205 sind die elektrischen Verbindungen der Komponenten, die auf dem Trägermaterial 205 angeordnet sind, ausgestaltet.

Durch die Elektronikeinheit 201 wird eine Kapazität jeweils zwischen den segmentierten Messelektroden 201a bestimmt. Der Füllstand kann aus einem Vergleich der Kapazitäten zwischen den einzelnen Segmenten bestimmt werden.

Der Füllstand kann von der Elektronikeinheit 201 über das standardisierte Interface 203 an das Steuergerät (100 in Fig. 1 und 2) abgegeben werden.

Die Füllstandsmessanordnung 200 ist weiterhin durch den Befestigungsmechanismus 205 mit dem Steuergerät verbindbar ausgestaltet. Der Befestigungsmechanismus 205 umfasst beispielsweise Pins, die in das Steuergerät gesteckt werden können.

Nicht dargestellt aber ebenfalls enthalten sein können die in Fig. 5a gezeigten Referenzelektroden.

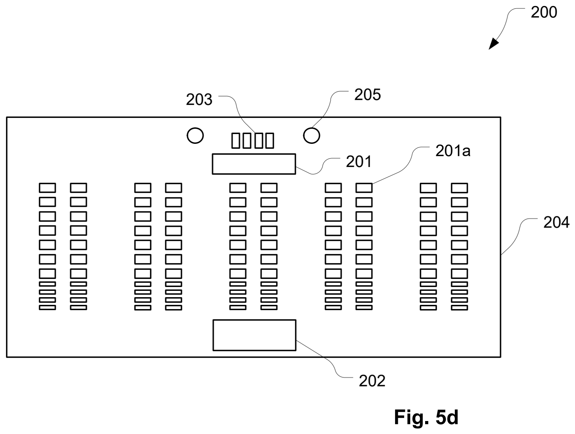

Fig. 5d zeigt eine Füllstandsmessanordnung nach einem Ausführungsbeispiel.

Die Füllstandsmessanordnung 200 umfasst eine Elektronikeinheit 201, segmentierte Messelektroden 201a und ein standardisiertes Interface 203 auf einem Trägermaterial 204 und einen Befestigungsmechanismus 205. Es ist weiterhin der Aktuator 202 umfasst.

Das Trägermaterial 205 kann ein flexibles, steifes oder gedrucktes Printed Circuit Board sein. In dem Trägermaterial 205 sind die elektrischen Verbindungen der Komponenten, die auf dem Trägermaterial 205 angeordnet sind, ausgestaltet.

Durch die Elektronikeinheit 201 wird eine Kapazität jeweils zwischen den segmentierten Messelektroden 201a bestimmt. Der Füllstand kann aus einem Vergleich der Kapazitäten zwischen den einzelnen Segmenten, die auf gleicher Höhe angeordnet sind, bestimmt werden. Es sind mehrere Spalten von Messelektroden 201a ausgestaltet, die den Füllstand in mehrere Spalten in einem korrespondierenden Behälter (300 in Fig. 1) bestimmen können.

Der Füllstand kann von der Elektronikeinheit 201 über das standardisierte Interface 203 an das Steuergerät (100 in Fig. 1 und 2) abgegeben werden.

Die Füllstandsmessanordnung 200 ist weiterhin durch den Befestigungsmechanismus 205 mit dem Steuergerät verbindbar ausgestaltet. Der Befestigungsmechanismus 205 umfasst beispielsweise Pins, die in das Steuergerät gesteckt werden können.

Der Aktuator 202 kann von der Elektronikeinheit 201 oder dem Steuergerät (100 in Fig. 2) angesteuert werden, um ein Ablassventil des Behälters zu bedienen.

Nicht dargestellt aber ebenfalls enthalten sein können die in Fig. 5a gezeigten Referenzelektroden.

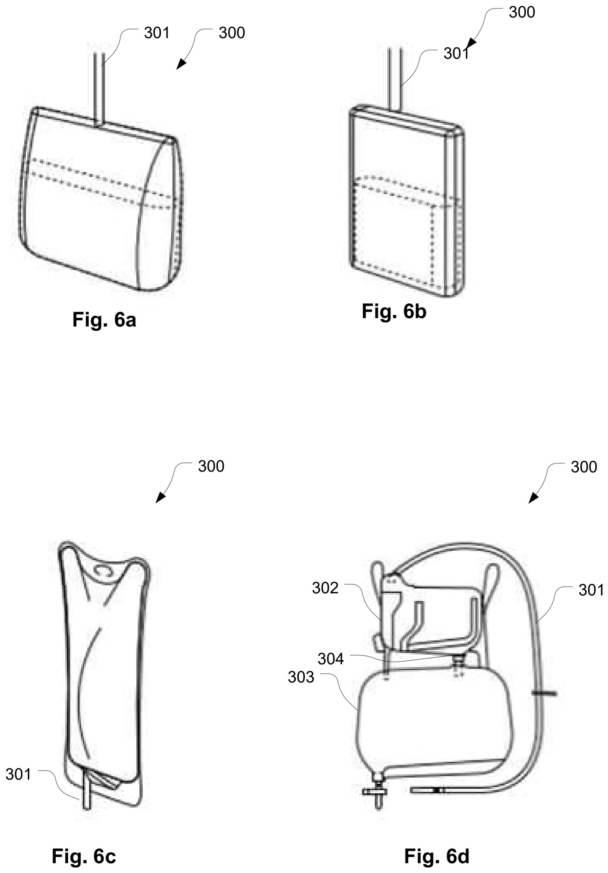

Fig. 6a zeigt einen Behälter nach einem Ausführungsbeispiel.

Der Behälter 300 ist mit einem Schlauch 301 als Zulauf ausgestaltet. In der dargestellten Form ist der Behälter ein (Urin-)Beutel und dient dem Auffangen von Urin eines Patienten auf einer Normalstation eines Krankenhauses oder einer Pflegeeinrichtung.

Fig. 6b zeigt einen Behälter nach einem Ausführungsbeispiel.

Der Behälter 300 ist mit einem Schlauch 301 als Zulauf ausgestaltet. In der dargestellten Form ist der Behälter eine Drainage und dient dem Auffangen von Fluiden aus einer Wunde eines Patienten, beispielsweise nach einer Operation des Patienten.

Fig. 6c zeigt einen Behälter nach einem Ausführungsbeispiel.

Der Behälter 300 ist mit einem Schlauch 301 als Ablauf ausgestaltet. In der dargestellten Form ist der Behälter ein Infusionsbeutel und dient der Bevorratung und geplanten Abgabe von Medikamenten, Nährstoffen, Mineralien in den Blutkreislauf eines Patienten.

Fig. 6d zeigt einen Behälter nach einem Ausführungsbeispiel.

Der Behälter 300 ist mit einem Schlauch 301 als Zulauf, einem oberen Behälter 302, einem unteren Behälter 303 und einem Übergang 304 ausgestaltet. Der obere Behälter dient der Messung des Füllstandes, wobei der obere Behälter mehrere Spalten aufweist, in denen sich Flüssigkeitssäulen ansammeln können, die jeweils in die nächste Spalte überlaufen können. Eine Füllstandsmessung kann dann mit den mehreren Spalten an Elektroden der Füllstandsmessanordnung nach Fig. 5d erfolgen. Übergang 304 umfasst ein Ventil, das von dem Aktuator (202 in Fig. 5d) bedient wird. Dieses Ventil kann ein Quetschventil sein, welches an dem Behälter 300 lediglich einen flexiblen Schlauch benötigt. Der Aktuator quetscht den Schlauch, um das Ventil zu schließen. In der dargestellten Form ist der Behälter ein Urinmesssystem und dient dem Auffangen von Urin eines Patienten auf einer Intensivstation eines Krankenhauses.

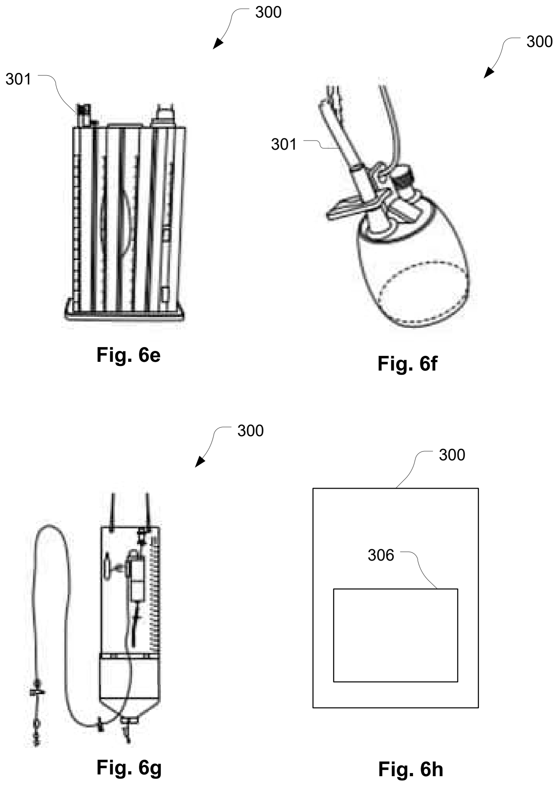

Fig. 6e zeigt einen Behälter nach einem Ausführungsbeispiel.

Der Behälter 300 ist mit einem Schlauch 301 als Zulauf ausgestaltet. In der dargestellten Form ist der Behälter eine Thoraxdrainage und dient dem Auffangen von Fluiden aus einer Wunde eines Patienten, beispielsweise nach einer Operation des Patienten. Der Behälter weist mehrere Spalten auf, in denen sich Flüssigkeitssäulen ansammeln können, die jeweils in die nächste Spalte überlaufen können. Eine Füllstandsmessung kann dann mit den mehreren Spalten an Elektroden der Füllstandsmessanordnung nach Fig. 5d erfolgen.

Fig. 6f zeigt einen Behälter nach einem Ausführungsbeispiel.

Der Behälter 300 ist mit einem Schlauch 301 als Zulauf ausgestaltet. In der dargestellten Form ist der Behälter eine chirurgische Drainage in Flaschenform und dient dem Auffangen von Fluiden aus einer Wunde eines Patienten, beispielsweise nach einer Operation des Patienten.

Fig. 6g zeigt einen Behälter nach einem Ausführungsbeispiel.

Der Behälter 300 ist mit einem Schlauch 301 als Zulauf ausgestaltet. In der dargestellten Form ist der Behälter eine Ventrikeldrainage und dient dem Auffangen von Gehirnwasser eines Patienten, beispielsweise nach einer Operation des Patienten.

Fig. 6h zeigt schematisch einen Behälter nach einem Ausführungsbeispiel.

Der Behälter 300 weist zusätzlich eine RFID-Vorrichtung 306 auf, die auf einer Oberfläche des Behälters 300 aufgebracht (angeklebt) sein kann. Die RFID-Vorrichtung 306 kann ein NFC-Tag sein und kann Patientendaten sowie einen Benutzungsstatus (ob der Behälter 300 bereits benutzt ist) speichern. Ferner kann die RFID-Vorrichtung 306 auch eine ID aussenden, wobei der Benutzungsstatus beispielsweise in einem Server (401 in Fig. 1), Steuergerät (100 in Fig. 1) oder Endgerät (402 in Fig. 1) für diese ID hinterlegt ist.

Die RFID-Vorrichtung 306 kann auf jedem der Behälter 300 aus Fig. 5a bis 5g aufgebracht sein.

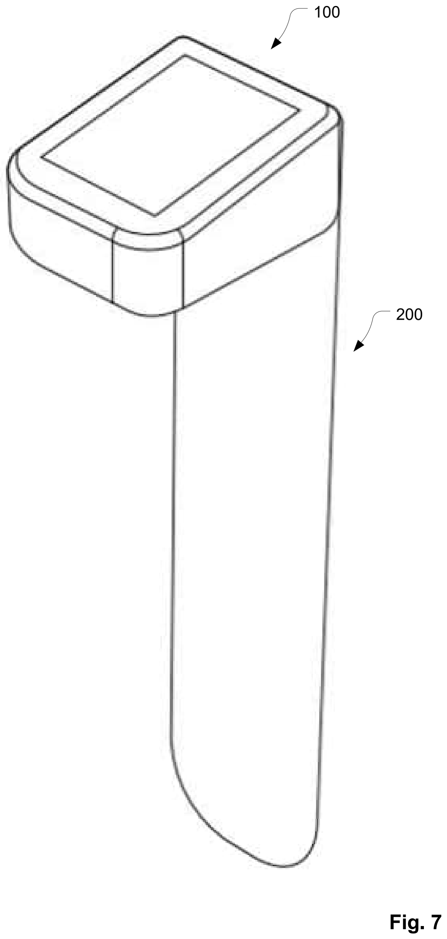

Fig. 7 zeigt ein Steuergerät und eine Füllstandsmessanordnung nach einem Ausführungsbeispiel.

Die Füllstandsmessanordnung 200 ist rückseitig mit dem Steuergerät 100 verbunden. Diese Verbindung ist beispielsweise durch einen Befestigungsmechanismus und ein standardisiertes Interface ausgestaltet. Die dargestellte Füllstandsmessanordnung 200 ist wie in Fig. 4a ausgestaltet und das dargestellte Steuergerät 100 wie in Fig. 3a.

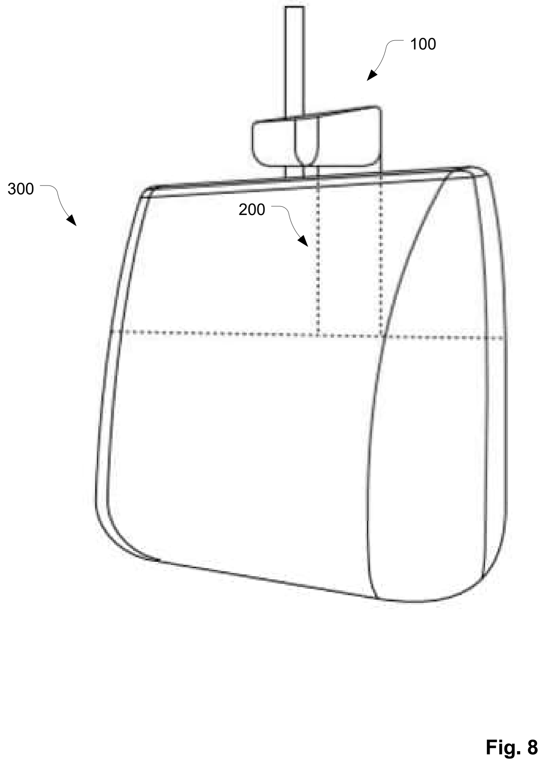

Fig. 8 zeigt ein Steuergerät, eine Füllstandsmessanordnung und einen Behälter nach einem Ausführungsbeispiel.

An dem Behälter 300 ist die Füllstandsmessanordnung 200 und das Steuergerät 100, die wie in Fig. 7 dargestellt ausgeführt sind, dargestellt. Der Behälter ist wie in Fig. 6a ausgeführt.

Die Füllstandsmessanordnung 200 liegt an der Rückseite des Behälters 300 an. Die Verbindung zwischen Behälter 300 und Füllstandsmessanordnung 200 kann eine Klebeverbindung sein.

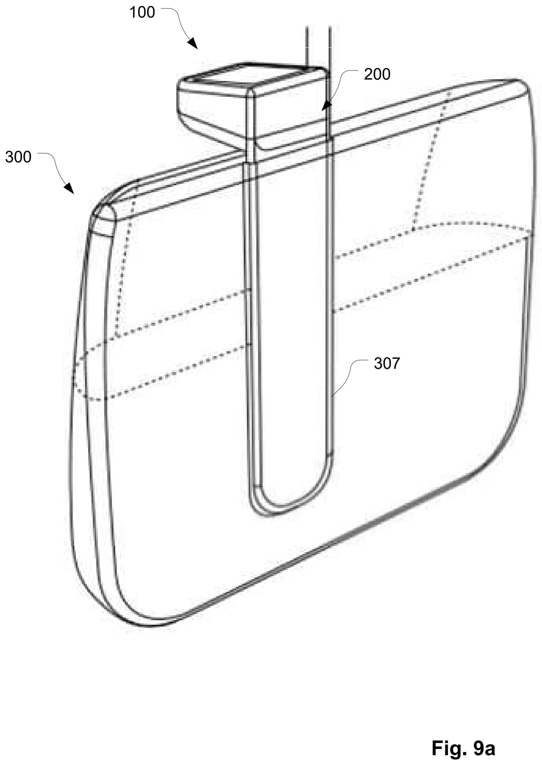

Fig. 9a zeigt ein Steuergerät, eine Füllstandsmessanordnung und einen Behälter nach einem Ausführungsbeispiel.

An dem Behälter 300 ist die Füllstandsmessanordnung 200 und das Steuergerät 100, die wie in Fig. 7 dargestellt ausgeführt sind, dargestellt. Der Behälter ist wie in Fig. 6a ausgeführt.

Die Füllstandsmessanordnung 200 steckt in einer flexiblen Tasche 307 des Behälters 300. Die Füllstandsmessanordnung 200 und die Tasche 307 sind nach dem Schwert-Scheide-Prinzip verbunden und somit liegt die Füllstandsmessanordnung 200 an der Rückseite des Behälters 300 an.

Die Tasche 307 kann aus demselben Material bestehen, aus dem die Außenwand des Behälters 300 besteht und mit der Behälteraußenwand verschweißt sein.

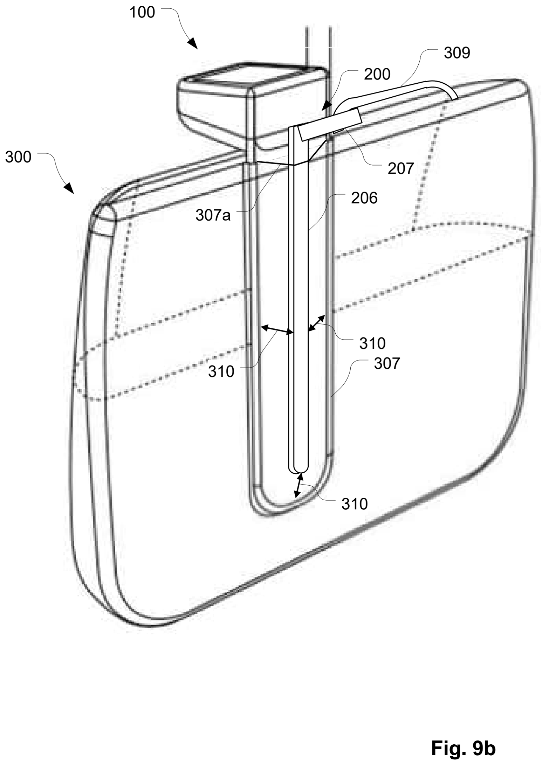

Fig. 9b zeigt ein Steuergerät, eine Füllstandsmessanordnung und einen Behälter nach einem Ausführungsbeispiel.

Das Steuergerät 100 ist wie in Fig. 9a ausgeführt. Die Füllstandsmessanordnung 200 steckt in der Tasche 307 und umfasst zusätzlich zu der Ausführung wie in Fig. 9a dargestellt ein rückenseitiges Spannelement 206 und ein Einrastelement 207. Der Behälter 300 umfasst zusätzlich zu der Ausführung wie in Fig. 9a dargestellt ein als Tragevorrichtung ausgestaltetes Gegenlager 309.

Das Einrastelement 207 ist mit dem rückseitigen Spannelement 206 verbunden. Das Einrastelement 207 rastet in das Gegenlager 309 des Behälters 300 ein und drück dabei das rückenseitige Spannelement 206 in die Tasche 307. Das Einrastelement kann an seiner Oberfläche, dort wo es an dem Gegenlager 309 des Behälters 300 einrastet eine Mulde (nicht dargestellt) aufweisen, um das Einrasten zu gewährleisten. Das rückenseitige Spannelement 206 ist entlang der Rückenseite der Füllstandsmessanordnung 200 ausgestaltet. Das rückenseitige Spannelement 206 ist mit der Füllstandsmessvorrichtung 200 verbunden oder liegt an dieser an.

Durch die Dimensionierung des rückenseitigen Spannelementes 206 wird beim Einführen des rückenseitigen Spannelementes 206 zusammen mit der Füllstandsmessanordnung 200 in die Tasche 307 eine Spannkraft in der Folie der Tasche erzeugt, indem die Folie über das rückenseitige Spannelement 206 gespannt wird. Die Spannkraft, die durch die Pfeile 310 dargestellt ist, spannt die Folie, die hin zum Außenbereich des Behälters angeordnet ist. Die horizontale Spannkraft kann alleine durch die geometrische Form des rückenseitigen Spannelementes 206 erzeugt werden, indem der Umfang der Tasche kleiner ist als der Umfang, der nötig ist, um die Füllstandsmessanordnung 200 und das rückenseitige Spannelement 206 zu umspannen. Die Dehnung in dem Material der Tasche erzeugt die Spannkraft. Mit dem Einrastelement 207 kann zudem eine vertikale Spannkraft erzeugt werden, indem die Füllstandsmessanordnung 200 und das rückenseitige Spannelement 206 in die Taschen hineingedrückt werden.

Ferner wird durch die Spannkraft jedoch auch die Folie hin zum Innenbereich des Behälters 300 über die dortige Oberfläche der Füllstandsmessanordnung 200 gespannt. Durch die Spannkraft werden dort Luftblasen zwischen Folie und Füllstandsmessanordnung 200 vermieden. Solche Luftblasen weisen die Dielektrizitätskonstante von Luft auf, was bei einer kapazitiven Messmethode des Füllstandes in dem Behälter 300 eine isolierende Wirkung hat und somit die Messung stört. Somit ist die Füllstandsmessung verbessert.

Um das Abfließen der Luft aus möglichen Luftblasen zwischen Folie und Oberfläche der Füllstandsmessanordnung 200 weiter zu verbessern, kann die Oberfläche mit einer Strukturierung versehen sein, die Luftkanäle aufweist. Ferner kann die Füllstandsmessanordnung 200 auch Löcher aufweisen, die das Fließen von Luft aus den Luftblasen ermöglichen.

Dadurch, dass sich die Luft nun in der Tasche auf der Rückenseite der Füllstandsmessanordnung 200, auf der das rückenseitige Spannelement 206 ausgebildet ist, sammelt, ergibt sich auf der Rückenseite eine isolierende Wirkung durch die Luft mit der entsprechenden Dielektrizitätskonstante. Da diese Seite jedoch zu dem Außenbereich des Behälters 300 zeigt, soll aus diesem Bereich kein Einfluss auf die Messung bestehen. Eine Abschirmung dieses Bereiches mittels der Luft in der Tasche ist somit eine weitere Verbesserung der Messung. Dadurch können beispielsweise Einflüsse von einem Patientenbett, das sich in diesem Bereich befinden kann, abgeschirmt werden.

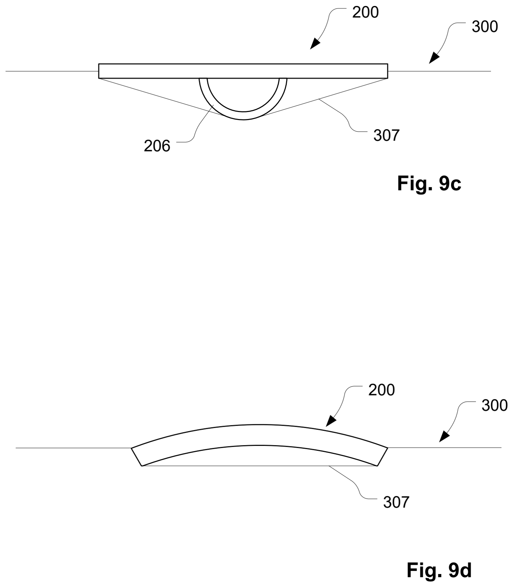

Fig. 9c zeigt einen Querschnitt durch die Tasche des Behälters mit der Füllstandsmessanordnung und der rückenseitigen Spannvorrichtung nach dem Ausführungsbeispiel aus Fig. 9b.

Dargestellt ist ein Ausschnitt aus der Wand des Behälters 300, wobei oberhalb der Wand das Innere des Behälters 300 ist und unterhalb der Wand der Außenbereich außerhalb des Behälters 300. Die Füllstandsmessanordnung 200 und die rückenseitigen Spannvorrichtung 206 sind in der Tasche 307 angeordnet. Die Folie ist über die rückenseitigen Spannvorrichtung 206 gespannt und liegt dadurch flach auf der Oberfläche der Füllstandsmessanordnung 200 an, wobei die Oberfläche, an der die Folie anliegt, hin zu dem Inneren des Behälters 300 weist.

Somit werden, wie mit Referenz zu Fig. 9b beschrieben, Luftblasen zwischen der Folie der Tasche 307 und der Oberfläche der Füllstandsmessanordnung 200 verdrängt. Die Folie der Tasche 307 ist dabei diejenige, die zwischen der Füllstandsmessanordnung 200 und der Innenseite des Behälters 300 liegt. Somit wird auch ein Luftpolster in der Tasche 307 zwischen der Füllstandsmessanordnung 200 und der Außenseite des Behälters 300 erzeugt. Ferner weist die rückenseitige Spannvorrichtung 206 einen halbrohrähnlichen Querschnitt auf, wodurch das Luftpolster auch innerhalb der rückenseitigen Spannvorrichtung 206 angeordnet ist. Somit wird eine Störung der Füllstandsmessung durch Luftblasen vermieden und der Außenbereich abgeschirmt.

Fig. 9d zeigt einen Querschnitt durch die Tasche des Behälters mit der Füllstandsmessanordnung nach einem Ausführungsbeispiel.

Alternative kann eine Spannkraft auch durch eine gebogene Füllstandsmessanordnung 200 erzeugt werden. Die Füllstandsmessanordnung 200 kann dabei auch flexibel sein und federn, wodurch die Spannkraft somit selbst erzeugen wird. Durch die Biegung der Füllstandsmessanordnung 200 wird die Folie hin zur Innenseite des Behälters 300 glatt über die Oberfläche der Füllstandsmessanordnung 200 gezogen, wodurch die Luftblasen zwischen diesen verdrängt werden. Weiterhin bildet sich durch die Biegung auch ein Luftpolster hin zur Außenseite des Behälters 300. Somit wird eine Störung der Füllstandsmessung durch Luftblasen vermieden und der Außenbereich abgeschirmt.

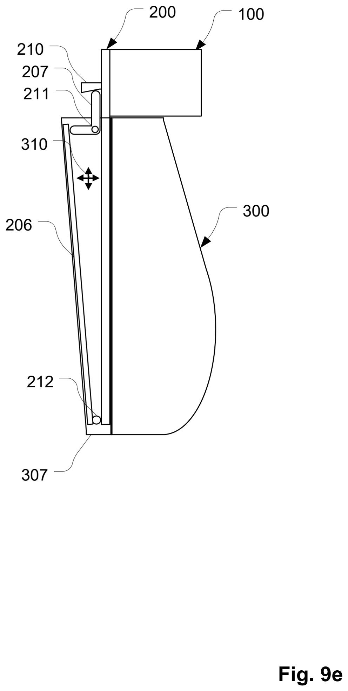

Fig. 9e zeigt ein Steuergerät, eine Füllstandsmessanordnung und einen Behälter nach einem Ausführungsbeispiel in einer Seitenansicht.

Das Steuergerät 100 ist wie in Fig. 9a ausgeführt. Die Füllstandsmessanordnung 200 steckt in der Tasche 307 und umfasst zusätzlich zu der Ausführung wie in Fig. 9a dargestellt ein rückenseitiges Spannelement 206, ein Einrastelement 207 und eine Einrastvorrichtung 210.

Das Spannelement 206 ist fußseitig drehbar (mittels eines Drehgelenks 212) an der Füllstandsmessanordnung 200 gelagert. Das Einrastelement 207 ist als drehbarer Hebel (dreht um Drehgelenks 211) ausgestaltet und kann durch das Umlegen die Spannkraft 310 erzeugen bzw. variieren.

Das Einrastelement 207 kann das rückenseitige Spannelement 206 von der Füllstandsmessanordnung 200 abspreizen, sodass die Tasche 307 durch den Abstand von dem rückenseitigen Spannelement 206 von der Füllstandsmessanordnung 200 gedehnt wird, wodurch die Spannkraft erzeugt wird.

Das als Hebel ausgeführte Einrastelement 207 ermöglicht das einfache, spannungsfreie Einschieben der Füllstandsmessanordnung 200 in die Tasche 307 und den Aufbau der Spannkraft durch das Umlegen des Einrastelements 207 in einer eingeschobenen Endposition.

Bei dem Umlegen des Einrastelement 207 rastet diese in einer Endposition der Umlegebewegung in der Einrastvorrichtung 210 ein. Die Einrastvorrichtung 210 kann an der Füllstandsmessanordnung 200, dem Steuergerät 100 oder dem Behälter ausgestaltet sein.

Ist die Einrastvorrichtung 210 an dem Behälter 300 ausgestaltet, wird verhindert, dass das Einrastelement 207 vor Erreichen der eingeschobenen Endposition umgelegt wird, da das Einrastelement 207 nur in dieser Endposition in der Einrastvorrichtung 210 eingerastet werden kann, wodurch auch eine vertikale Spannkraft (310) aufgebaut wird.

Dabei kann die Einrastvorrichtung 210 als eine Form von Gegenlager am Behälter 300 ausgestaltet sein, beispielsweise auch das als Tragevorrichtung ausgestaltete Gegenlager 309 wie in Fig. 9b. Dabei erzeugt das Gegenlager die vertikale Spannkraft 310.

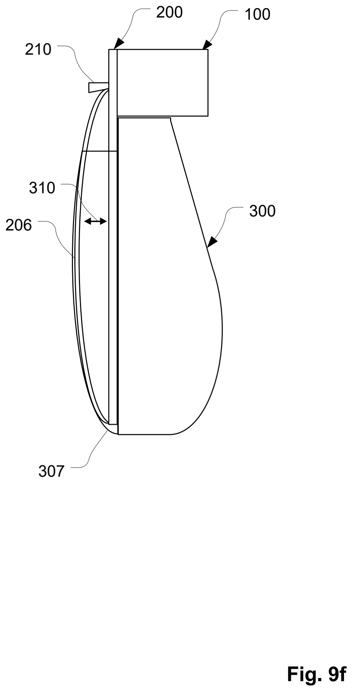

Fig. 9f zeigt ein Steuergerät, eine Füllstandsmessanordnung und einen Behälter nach einem Ausführungsbeispiel in einer Seitenansicht.

Im Vergleich zu dem Ausführungsbeispiel in Fig. 9e ist das Spannelement 206 biegbar ausgestaltet und erfüllt auch die Funktion des Einrastelementes, wodurch das Einrastelement in dem Spannelement 206 umfasst ist.

Das Spannelement 206 ist fußseitig an der Füllstandsmessanordnung 200 angeordnet (kann dort mittels eines Drehgelenks gelagert sein).

Wenn die Füllstandsmessanordnung 200 zusammen mit dem Spannelement 206 in die Tasche 307 eingeführt wird, verlaufen Füllstandsmessanordnung 200 und Spannelement 206 nahe beieinander (substanziell parallel), beispielsweise sodass die Tasche 307 nicht gedehnt wird. Ist einer eingeschobenen Endposition der Füllstandsmessanordnung 200 in der Tasche 307 erreicht, kann das biegbare Spannelement 206 weiter in die Tasche hineingeschoben werden. Dabei entfernt sich das Spannelement 206 von der Füllstandsmessanordnung 200, beispielsweise indem es einen Bogen formt. Somit ist in dem gebogenen Zustand des biegbaren Spannelementes 206 durch den Abstand des biegbaren Spannelementes 206 von der Füllstandsmessanordnung 200 die Tasche gedehnt und eine Spannkraft 310 erzeugt.

Das obere freie Ende des Spannelementes 206 wird dann in der Einrastvorrichtung 210 eingerastet. Die Einrastvorrichtung 210 kann an der Füllstandsmessanordnung 200, dem Steuergerät 100 oder dem Behälter ausgestaltet sein.

Ist die Einrastvorrichtung 210 an dem Behälter 300 ausgestaltet, wird auch eine vertikale Spannkraft (310) aufgebaut. Dabei kann die Einrastvorrichtung 210 als eine Form von Gegenlager am Behälter 300 ausgestaltet sein, beispielsweise auch das als Tragevorrichtung ausgestaltete Gegenlager 309 wie in Fig. 9b. Dabei erzeugt das Gegenlager die vertikale Spannkraft 310.

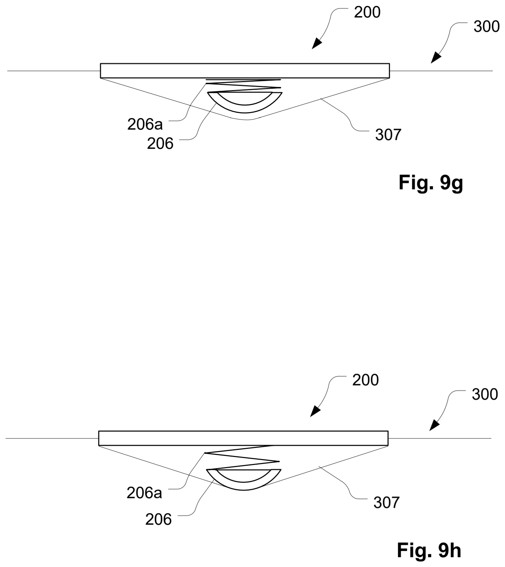

Fig. 9g einen Querschnitt durch die Tasche des Behälters mit der Füllstandsmessanordnung nach einem Ausführungsbeispiel.

Weiter alternative kann eine Spannkraft auch durch eine Federvorrichtung 206a erzeugt sein. Die Federvorrichtung 206a kann in der rückenseitigen Spannvorrichtung 206 umfasst sein. Die rückenseitige Spannvorrichtung 206 kann in einem Zustand, in dem die Federvorrichtung 206a komprimiert und somit vorgespannt ist, in die Tasche 307 eingeführt oder herausgenommen werden. Dies ist durch die reduzierte Höhe der rückeinseitigen Spannvorrichtung 206 über der Füllstandsmessanordnung 200 erreichbar. Fig. 9e zeigt den komprimierten Zustand. Die Tasche 307 liegt nicht an der rückenseitigen Spannvorrichtung 206 an und eine Spannkraft wird in diesem Zustand nicht erzeugt.

Fig. 9h einen Querschnitt durch die Tasche des Behälters mit der Füllstandsmessanordnung nach dem Ausführungsbeispiel aus Fig. 9g.

Sobald beim Einsetzen die Zielposition der rückenseitigen Spannvorrichtung 206 erreicht ist, kann die Federvorrichtung 206a dekomprimiert werden, dabei expandiert die Federvorrichtung 206a und drückt die Spannvorrichtung 206 gegen die Innenwand der Tasche 307. Die Fig. 9h zeigt den dekomprimierten Zustand der Federvorrichtung 206a, in dem die Spannkraft in der Tasche 307 durch die Federvorrichtung 206a erzeugt ist.

Die Federvorrichtung 206a kann, beispielsweise durch einen Nutzer, per Hand komprimiert werden. Beispielsweise in dem auf die rückenseitige Spannvorrichtung 206 gedrückt wird. Weiterhin kann ein Haltemechanismus (nicht dargestellt) vorgesehen sein, der die rückenseitige Spannvorrichtung 206 in einer Position hält, in der die Federvorrichtung 206a komprimiert ist. Dieser Haltemechanismus kann einen separaten Betätigungsmechanismus aufweisen, mit dem der Nutzer den Haltemechanismus lösen kann und somit die rückenseitige Spannvorrichtung 206 aus der gehaltenen komprimierten Position ausfedern kann. Dieser Betätigungsmechanismus kann an ein Einrastelement (wie 207 in Fig. 9b) gekoppelt sein. Sobald das Einrastelement an einer in der Tragevorrichtung (wie 309 in Fig. 9b) des Behälters 300 einrastet, wird der Haltemechanismus gelöst und somit kann die rückenseitige Spannvorrichtung 206 aus der gehaltenen komprimierten Position ausfedern.

Zum Entnehmen der rückenseitigen Spannvorrichtung 206, der Federvorrichtung 206a und der Füllstandsmessanordnung 200 kann das Spannelement 207 gegen die Füllstandsmessanordnung 200 gedrückt werden, wodurch die Federvorrichtung 206a komprimiert wird. Die rückenseitige Spannvorrichtung 206 und die Federvorrichtung 206a können in dieser Position durch den Haltemechanismus gehalten werden, wodurch die Entnahme der rückenseitigen Spannvorrichtung 206, der Federvorrichtung 206a und der Füllstandsmessanordnung 200 aus der Tasche 307 einfacher ist.

Der beschriebene Mechanismus könnte statt der Federvorrichtung 206a auch über Aktuatoren (elektrisch, magnetisch, pneumatisch, hydraulisch, piezoelektrisch etc.) angetrieben werden, da die Füllstandsmessanordnung 200 über eine eigene Stromversorgung verfügt.

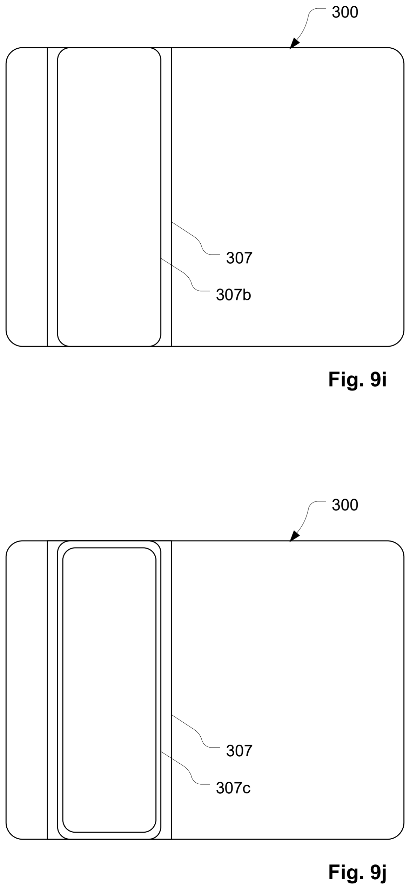

Fig. 9i zeigt eine Rückseite des Behälters nach einem Ausführungsbeispiel.

Es ist die Rückseite des Behälters 300 von der Außensite des Behälters 300 dargestellt. Der Behälter umfasst ein Tasche 307 und eine Versteifung 307b in der Tasche 307. Die Versteifung 307b ist in der Tasche auf der Folie hin zum Innenbereich des Behälters 300 ausgeformt. Somit liegt die Versteifung 307b bei eingeführter Füllstandsmessanordnung (nicht dargestellt) zwischen der Füllstandsmessanordnung und dem Behälterinneren. Die Versteifung 307b kann auf die Folie des Behälters 300 aufgebracht sein oder die Folie des Behälters 300 kann zumindest in der Tasche 307 dicker ausgestaltet sein, um die Versteifung 307b zu erzeugen.

Durch die Versteifung 307b werden Falten vermieden, die Luftblasen zwischen der Folie hin zum Innenbereich des Behälters 300 und Füllstandsmessanordnung erzeugen können. Solche Luftblasen weisen die Dielektrizitätskonstante von Luft auf, was bei einer kapazitiven Messmethode des Füllstandes in dem Behälter 300 eine isolierende Wirkung hat und somit die Messung stört. Somit ist die Füllstandsmessung verbessert.

Fig. 9j zeigt eine Rückseite des Behälters nach einem Ausführungsbeispiel.

Es ist die Rückseite des Behälters 300 von der Außensite des Behälters 300 dargestellt. Der Behälter umfasst ein Tasche 307 und eine Versteifung 307c in der Tasche 307. Im Vergleich zu der Versteifung 307b aus Fig. 9i ist die Versteifung 307c als Rahmen ausgestaltet. Die Versteifung 307c kann die Folie hin zum Innenbereich des Behälters 300 spannen und somit Luftblasen zwischen einer Füllstandsmessanordnung (nicht dargestellt) und der Folie hin zum Innenbereich des Behälters 300 vermeiden. Die Versteifung 307c kann dabei einen Kontaktbereich eines Elektrodenarrays der Füllstandsmessanordnung umlaufen, und dabei eine Spannkraft aufbauen, welche die Folie der Beutelrückwand im Inneren glattzieht, um Falten in welchen sich Luftblasen sammeln können zu vermeiden.

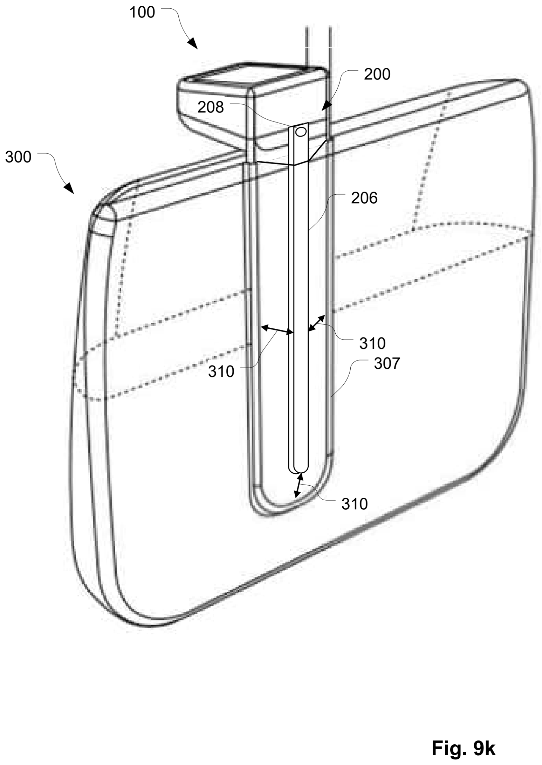

Fig. 9k zeigt ein Steuergerät, eine Füllstandsmessanordnung und einen Behälter nach einem Ausführungsbeispiel.

Das Ausführungsbeispiel nach Fig. 9g ist eine Modifikation des Ausführungsbeispiels nach Fig. 9b. Hierbei weist das rückenseitige Spannelement 206 ein Rückschlagventil (nicht dargestellt) und einen Anschluss 208 auf und ist über diese aufblasbar, um die Spannkraft innerhalb der Tasche 307 zu erzeugen. Zum Aufblasen kann beispielsweise eine Pumpe oder eine Spritze mit dem Anschluss verbunden werden. Beim Aufblasen dehnt sich die rückseitige Spannvorrichtung aus und erzeugt somit die Streckung des Materials der Tasche 307, wodurch die Spannkraft erzeugt wird. Das Rückschlagventil hält den Druck im Inneren der rückenseitigen Spannvorrichtung 206. Die aufblasbare rückenseitige Spannvorrichtung 206 kann auch mit einem Einrastelement wie in Fig. 9b dargestellt kombiniert werden.

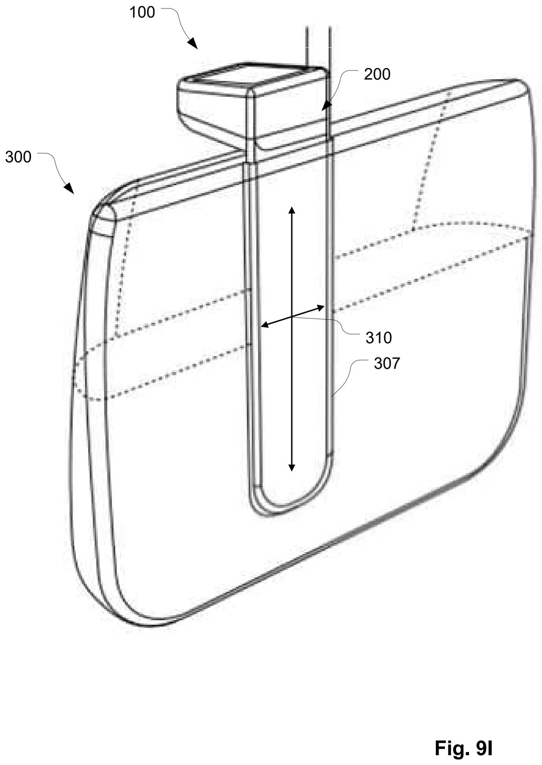

Fig. 9l zeigt ein Steuergerät, eine Füllstandsmessanordnung und einen Behälter nach einem Ausführungsbeispiel.

Die Tasche 307 ist aus einem elastischen Material gefertigt und so dimensioniert, dass eine Spannkraft erzeugt wird, wenn die Füllstandsmessanordnung 200 in die Tasche 307 eingeführt wird und diese dabei dehnt.

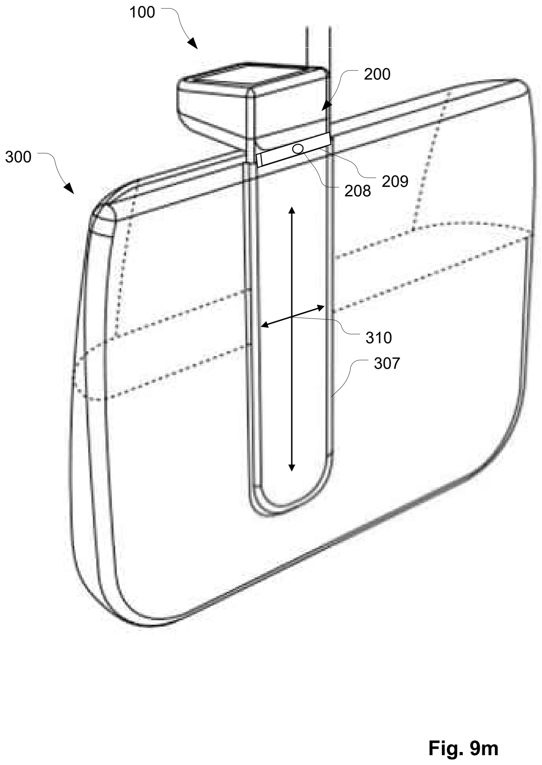

Fig. 9m zeigt ein Steuergerät, eine Füllstandsmessanordnung und einen Behälter nach einem Ausführungsbeispiel.

An der Füllstandsmessanordnung 200 ist ein Abdichtungsbereich 209 ausgeführt, der die Tasche abdichtet, sodass über den Anschluss 208 und ein Rückschlagventil (nicht dargestellt) Luft aus der Tasche gesaugt werden kann. Zum Absaugen der Luft kann beispielsweise eine Pumpe oder eine Spritze mit dem Anschluss 208 verbunden werden.

Bei rückenseitigen Spannvorrichtungen, die separat zu der Füllstandsmessanordnung 200 in die Tasche eingeführt werden können, ist es zudem so, dass die Reibung von Füllstandsmessanordnung 200 und rückenseitiger Spannvorrichtung mit dem Inneren der Tasche 307 durch das separate Einführen oder bei der Demontage das separate Rausziehen vereinfacht wird.

Ein analoger Effekt zu der verbesserten Messung, die mit Referenz zu Fig. 9b bis m beschrieben ist, kann ebenfalls für eine Füllstandsmessanordnung 200 erreicht werden, die als Elektrodenarrays auf den Behälter 300 aufgedruckt sind, z.B. mit elektronischer Tinte oder als Wegwerfsensoren, auf den auf den Behälter aufgeklebt sind. Durch das Kleben oder Ducken können die Luftblasen bereits vermieden werden, allerdings zulasten einer Wiederverwendbarkeit der Füllstandsmessanordnung 200, die dann mit dem Behälter entsorgt wird. Die Abschirmung des Außenbereichs kann beispielsweise durch ein Aufkleben eines luftenthaltenden Streifens, beispielsweise eines Schaumstreifens oder Luftpolsterfolie auf der Außenseite der Füllstandsmessanordnung 200 an dem Behälter 300 erreicht werden.

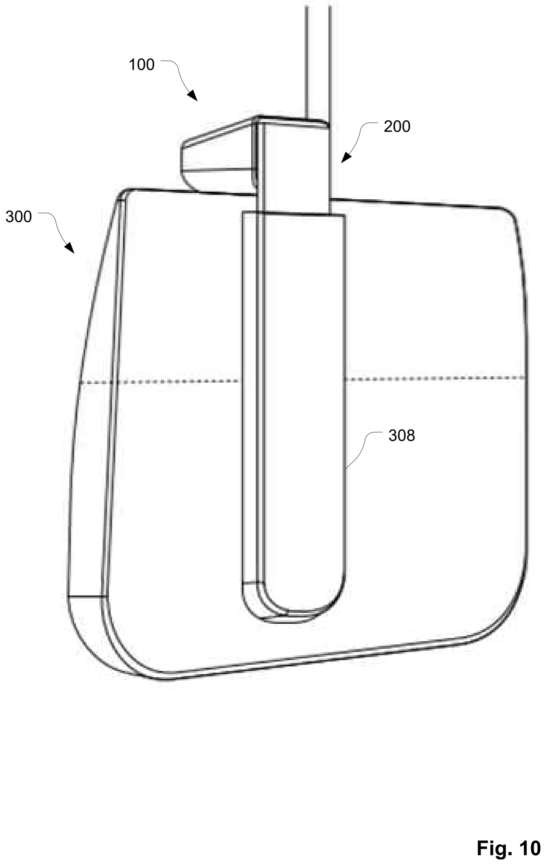

Fig. 10 zeigt ein Steuergerät, eine Füllstandsmessanordnung und einen Behälter nach einem Ausführungsbeispiel.

An dem Behälter 300 ist die Füllstandsmessanordnung 200 und das Steuergerät 100, die wie in Fig. 7 dargestellt ausgeführt sind, dargestellt. Der Behälter 300 ist wie in Fig. 6a ausgeführt.

Die Füllstandsmessanordnung 200 steckt in einer steifen Tasche 308 des Behälters 300. Die Füllstandsmessanordnung 200 und die Tasche 308 sind nach dem Schwert-Scheide-Prinzip verbunden und somit liegt die Füllstandsmessanordnung 200 an der Rückseite des Behälters 300 an.

Die Tasche 308 kann aus einem festen Kunststoff bestehen und mit der Behälteraußenwand verklebt sein, wobei die Tasche der Behälterwand eine Steifigkeit verleiht, die die Messgenauigkeit der Füllstandsmessung aufgrund einer geringeren Wölbung der Behälterwand verbessert.

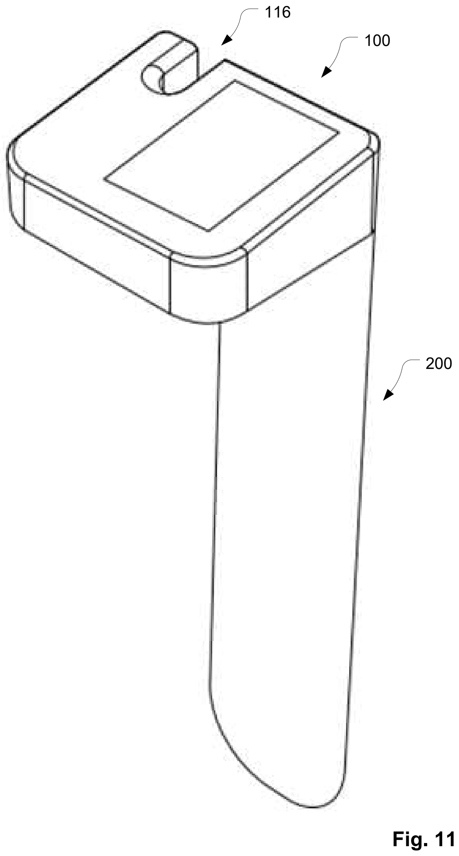

Fig. 11 zeigt ein Steuergerät und eine Füllstandsmessanordnung nach einem Ausführungsbeispiel.

Die Füllstandsmessanordnung 200 ist rückseitig mit dem Steuergerät 100 verbunden. Diese Verbindung ist beispielsweise durch einen Befestigungsmechanismus und ein standardisiertes Interface ausgestaltet. Die dargestellte Füllstandsmessanordnung 200 ist wie in Fig. 4a ausgestaltet und das dargestellte Steuergerät 100 wie in Fig. 3b mit einer Diagnosevorrichtung 116.

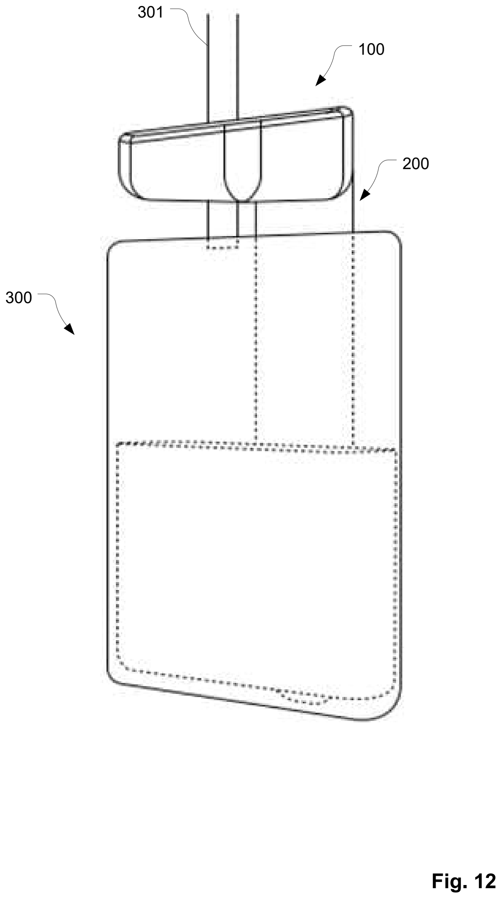

Fig. 12 zeigt ein Steuergerät, eine Füllstandsmessanordnung und einen Behälter nach einem Ausführungsbeispiel.

An dem Behälter 300 ist die Füllstandsmessanordnung 200 und das Steuergerät 100, die wie in Fig. 11 dargestellt ausgeführt sind, dargestellt. Der Behälter ist wie in Fig. 6b ausgeführt.

Die Füllstandsmessanordnung 200 liegt an der Rückseite des Behälters 300 an. Die Verbindung zwischen Behälter 300 und Füllstandsmessanordnung 200 kann eine Klebeverbindung sein.

Die Füllstandsmessanordnung 200 und der Behälter 300 können auch über eine flexible Tasche an der Rückwand des Behälters verbunden sein, so wie diese Verbindung bereits in Fig. 9 dargestellt ist.

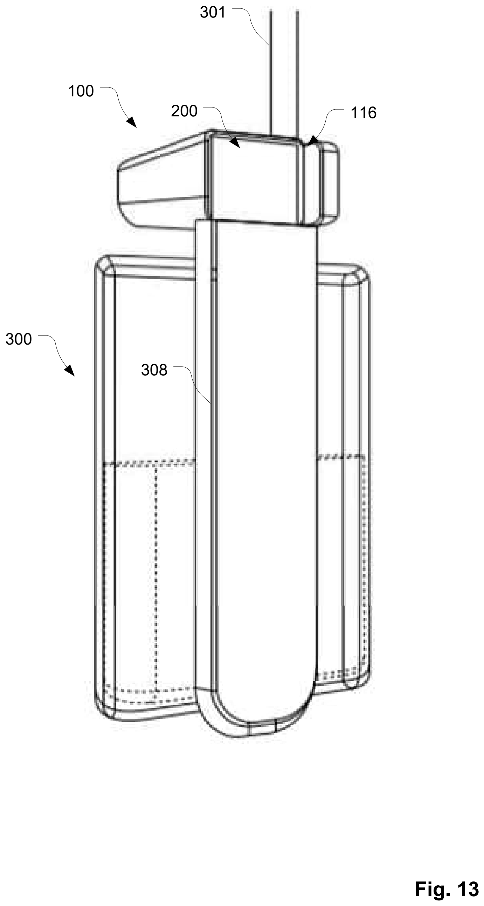

Fig. 13 zeigt ein Steuergerät, eine Füllstandsmessanordnung und einen Behälter nach einem Ausführungsbeispiel.

An dem Behälter 300 ist die Füllstandsmessanordnung 200 und das Steuergerät 100, die wie in Fig. 11 dargestellt ausgeführt sind, dargestellt. Der Behälter 300 ist wie in Fig. 6b ausgeführt.

Die Füllstandsmessanordnung 200 steckt in einer steifen Tasche 308 des Behälters 300. Die Füllstandsmessanordnung 200 und die Tasche 308 sind nach dem Schwert-Scheide-Prinzip verbunden und somit liegt die Füllstandsmessanordnung 200 an der Rückseite des Behälters 300 an.

Die Tasche 308 kann aus einem festen Kunststoff bestehen und mit der Behälteraußenwand verklebt sein, wobei die Tasche der Behälterwand eine Steifigkeit verleiht, die die Messgenauigkeit der Füllstandsmessung aufgrund einer geringeren Wölbung der Behälterwand verbessert.

Der Schlauch 301 des Behälters 300 wird in der verbundenen Position des Behälters 300 mit der Füllstandsmessanordnung 200 durch die Diagnosevorrichtung 116 geführt. Somit kann Fluid, das durch den Schlauch 301 fließt, von der Diagnosevorrichtung 116 analysiert werden.

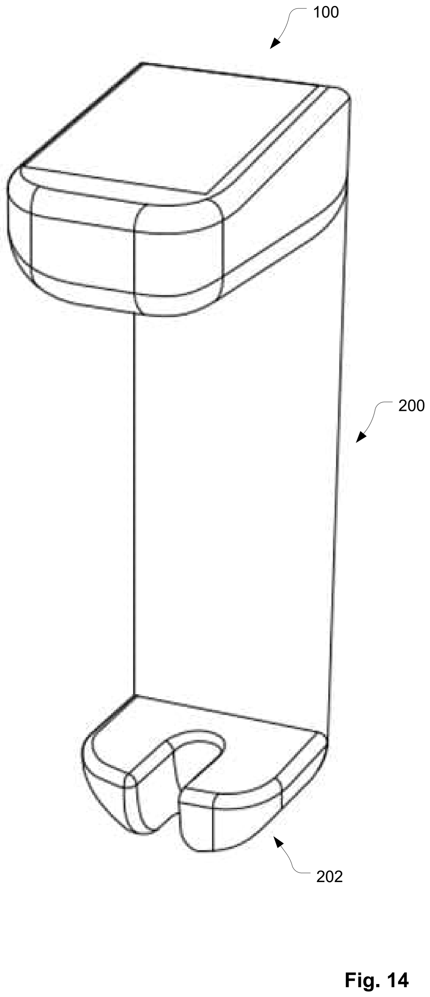

Fig. 14 zeigt ein Steuergerät und eine Füllstandsmessanordnung nach einem Ausführungsbeispiel.

Die Füllstandsmessanordnung 200 ist rückseitig mit dem Steuergerät 100 verbunden. Diese Verbindung ist beispielsweise durch einen Befestigungsmechanismus und ein standardisiertes Interface ausgestaltet. Die dargestellte Füllstandsmessanordnung 200 ist wie in Fig. 4b ausgestaltet und das dargestellte Steuergerät 100 wie in Fig. 3a. Die Füllstandsmessanordnung 200 weist eine Halterung mit Aktuator 202 auf.

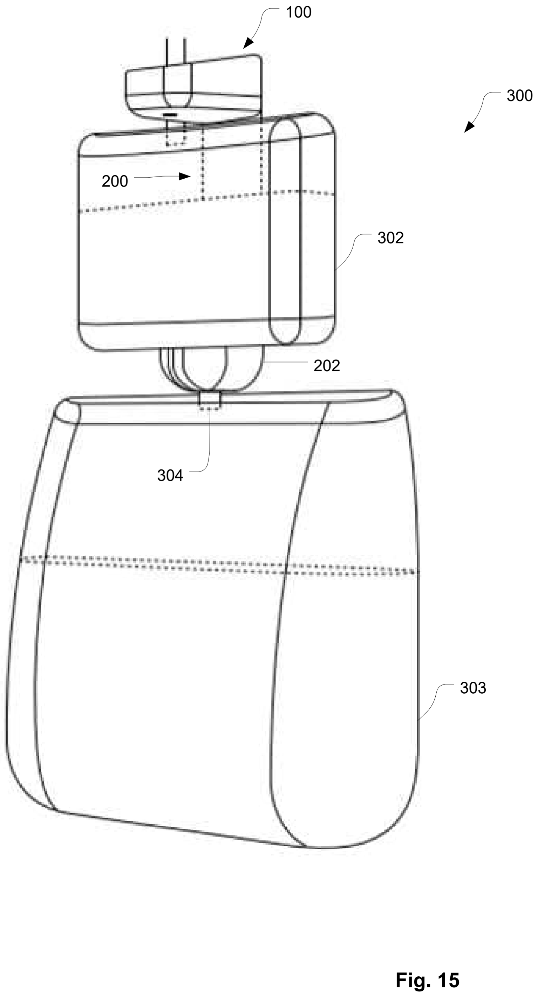

Fig. 15 zeigt ein Steuergerät, eine Füllstandsmessanordnung und einen Behälter nach einem Ausführungsbeispiel.

An dem Behälter 300 ist die Füllstandsmessanordnung 200 und das Steuergerät 100, die wie in Fig. 14 dargestellt ausgeführt sind, dargestellt. Der Behälter 300 ist wie in Fig. 6d ausgeführt.

Die Halterung mit Aktuator 202 der Füllstandsmessanordnung 200 hält den Behälter 300 an dem Übergang 304 zwischen oberem Behälter 302 und unterem Behälter 303. Die Füllstandsmessanordnung 200 liegt an der Rückseite des oberen Behälters 302 an und misst dort den Füllstand. Es kann in dem oberen Behälter auch der Füllstand mehrerer Spalten gemessen werden, so wie dies bereits mit Bezug auf Fig. 5d und 6d beschrieben wurde. Der Aktuator 202 kann den Übergang 304 verschließen, indem dieser gequetscht wird.

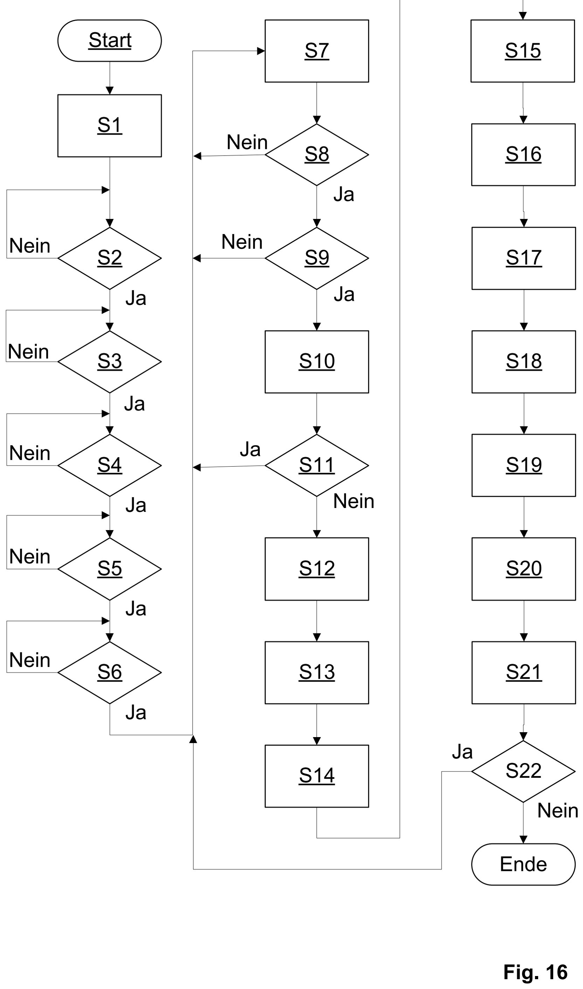

Fig. 16 zeigt ein Flussdiagramm einer beispielhaften Füllstandsmessung.

Die Füllstandsmessung wird von dem Steuergerät ausgeführt. Nach dem Start des Steuergerätes wird das System in Schritt S1 initialisiert. In Schritt S2 wird überprüft, ob und welche Füllstandsmessanordnung verbunden ist. Ist keine Füllstandsmessanordnung verbunden, wird der Schritt S2 wiederholt, dabei kann eine Fehlermeldung ausgegeben werden. Ist eine Füllstandsmessanordnung verbunden, folgt Schritt S3.

In Schritt S3 wird überprüft, ob das Steuergerät mit einem Netzwerk verbunden ist. Ist das Steuergerät nicht mit einem Netzwerk verbunden, wird der Schritt S3 wiederholt, dabei kann eine Fehlermeldung ausgegeben werden. Ist das Steuergerät mit einem Netzwerk verbunden, folgt Schritt S4.

In Schritt S4 wird überprüft, ob das Steuergerät mit einer elektronischen Patientenakte (beispielsweise auf dem Server 401 in Fig. 1) gekoppelt ist. Ist das Steuergerät nicht mit einer elektronischen Patientenakte gekoppelt, so wird Schritt S4 wiederholt, dabei kann eine Fehlermeldung ausgegeben werden. Ist das Steuergerät mit einer elektronischen Patientenakte gekoppelt so folgt Schritt S5.

In Schritt S5 wird überprüft, ob der Behälter erkannt wurde (anhand seines NFC-Tags). Ist der Behälter nicht erkannt worden, wird Schritt S5 wiederholt, dabei kann eine Fehlermeldung ausgegeben werden. Ist der Behälter erkannt worden, so folgt Schritt S6.

In Schritt S6 wird überprüft, ob der Behälter neu ist, ein zugelassener Behälter ist und mit der Füllstandsmessanordnung kompatibel. Zudem wird eine mathematische Umrechnungsfunktion geladen, die von der Geometrie von Behälter und Füllstandsmessanordnung abhängig ist, welche in den Schritten S12 bis S17 zur Konversion und Korrektur der Messdaten benötigt wird. Trifft dies nicht zu, so wird der Schritt S6 wiederholt, dabei kann eine Fehlermeldung ausgegeben werden. Trifft dies zu so folgt Schritt S7.

In Schritt S7 folg der Beginn der Messung. Dabei werden kapazitive Messwerte der Füllstandesmessungsanordnung erfasst, diese sind aber noch nicht freigegeben. Weiterhin werden Messwerte einer Inertial Measurement Unit (IMU) erfasst.

In Schritt S8 wird überprüft, ob die von der IMU gemessene Lage des Steuergerätes und somit auch des Behälters in einem Toleranzbereich um eine Referenzlage (senkrecht) liegt. Liegt die Lage nicht in dem Toleranzbereich, so folgt Schritt S7. Bei dem Sprung zu Schritt 7 kann eine Fehlermeldung ausgegeben werden. Liegt die gemessene Lage innerhalb des Toleranzbereiches so folgt Schritt S9.

Im Schritt S9 wird überprüft, ob die von der IMU gemessene Beschleunigung innerhalb eines Toleranzbereiches liegt. Liegt die Beschleunigung nicht innerhalb des Toleranzbereiches, so folgt Schritt S7. Bei dem Sprung zu Schritt 7 kann eine Fehlermeldung ausgegeben werden. Liegt die gemessene Beschleunigung innerhalb des Toleranzbereiches so folgt Schritt S10.

In Schritt S10 werden die Messwerte der Füllstandesmessungsanordnung erfasst und als Plausibilitätsmessung ausgewertet.

In Schritt S11 wird anhand der Plausibilitätsmessung überprüft, ob eine überhöhte Änderung des Füllstandes im Vergleich zu einem vorherig gemessenen Füllstand gemessen wurde (Vergleich der Änderung mit einem Schwellenwert). Wird eine überhöhte Änderung festgestellt, so folgt Schritt S7. Bei dem Sprung zu Schritt 7 kann eine Fehlermeldung ausgegeben werden. Wird keine überhöhte Änderung festgestellt, ergeht ein Freigabekriterium für die Messwerte der Füllstandesmessungsanordnung in der Plausibilitätsmessung.

In Schritt S12 werden die kapazitiven Messwerte aus der Plausibilitätsmessung in einen Füllstand umgerechnet. Dazu wird die in S6 geladene Umrechnungsfunktion verwendet, welche von der Geometrie der erkannten Behälter und Füllstandsmessanordnung abhängt.

In Schritt S13 wird der bestimmte Füllstand in ein Volumen konvertiert. Dazu wird die in S6 geladene Umrechnungsfunktion verwendet, welche von der Geometrie der erkannten Behälter und Füllstandsmessanordnung abhängt.

In Schritt S14 wird eine Abweichung der aus der IMU gemessenen Lage von der Referenzlage bestimmt. Dazu wird die in S6 geladene Umrechnungsfunktion verwendet, welche von der Geometrie der erkannten Behälter und Füllstandsmessanordnung abhängt.

In Schritt S15 wird das bestimmte Volumen aufgrund der bestimmten Abweichung korrigiert. Dazu wird die in S6 geladene Umrechnungsfunktion verwendet, welche von der Geometrie der erkannten Behälter und Füllstandsmessanordnung abhängt.

In Schritt S16 wird die Temperatur der Flüssigkeit und die Temperatur der Umgebung erfasst.

In Schritt S17 wird eine weitere Korrektur des korrigierten Volumens anhand der Temperaturmesswerte durchgeführt. Somit wird ein korrigiertes Fluidvolumen erhalten. Dazu wird die in S6 geladene Umrechnungsfunktion verwendet, welche von der Geometrie der erkannten Behälter und Füllstandsmessanordnung abhängt.

In Schritt S18 wird das korrigierte Fluidvolumen lokal auf dem Steuergerät gespeichert.

In Schritt S19 wird das korrigierte Fluidvolumen auf der Anzeige angezeigt.

In Schritt S20 wird das korrigierte Fluidvolumen an den Server übermittelt.

In Schritt S21 wird das korrigierte Fluidvolumen in die Patientenakte übermittelt (gespeichert).

In Schritt S22 wird überprüft, ob eine weitere Messung durchgeführt werden soll. Soll eine weitere Messung durchgeführt werden, so wird mit Schritt S7 fortgefahren. Soll keine weitere Messung durchgeführt werden, so endet die Messung.

Fig. 17 veranschaulicht ein Ausführungsbeispiel eines Mehrzweckcomputers 500. Der Mehrzweckcomputer 500 ist ein Beispiel für ein Steuergerät nach Fig. 2, welches eine Schaltung umfasst, die dazu eingerichtet ist, das Verfahren gemäß der vorliegenden Technologie (z. B. das Verfahren aus Fig. 17) auszuführen.

Ausführungsformen, welche Software, Firmware, Programme oder dergleichen verwenden, um die hierin beschriebenen Verfahren auszuführen, können auf dem Computer 500 installiert werden sein, welcher dann für das entsprechende Ausführungsbeispiel geeignet eingerichtet ist.

Der Computer 500 hat eine Zentraleinheit (Central Processing Unit, CPU) 501, welche mancherlei Arten von Prozeduren und Verfahren ausführen kann, wie hierin beschrieben, zum Beispiel gemäß Programmen, die in einem Festwertspeicher (Read-Only Memory, ROM) 502 gespeichert sind; die in einem Speicher 507 gespeichert sind und in einen Arbeitsspeicher (Random-Access Memory, RAM) 503 geladen werden; die auf einem Medium 510 gespeichert sind, welches in ein entsprechendes Laufwerk 509 eingeführt werden kann; etc.

Die CPU 501, der ROM 502 und der RAM 503 sind mit einem Bus 511 verbunden, welcher wiederum mit einer Eingabe-/Ausgabe-Schnittstelle 504 verbunden ist. Die Anzahl an CPUs, Arbeitsspeichern und Speichern ist nur exemplarisch und der Fachmann wird verstehen, dass der Computer 500 entsprechend angepasst und eingerichtet werden kann, damit er spezifische Anforderungen erfüllen kann, die entstehen, wenn er als Steuergerät gemäß der vorliegenden Technologie eingesetzt wird.

An der Eingabe-/Ausgabe-Schnittstelle 504 sind mehrere Komponenten verbunden: eine Eingabe 505, eine Ausgabe 506, ein Speicher 507, eine Kommunikationsschnittstelle 508 und das Laufwerk 509, in das ein Medium 510 (Compact Disk (CD), Digital Video Disc (DVD), CompactFlash-Speicher oder dergleichen) eingeführt werden kann.

Die Eingabe 505 kann ein Zeigergerät (Maus, Grafiktablett oder dergleichen), eine Tastatur, ein Mikrofon, eine Kamera, ein Berührungsbildschirm, eine Blickerfassungseinheit etc. umfassen. Die Eingabe kann weiterhin sämtliche Messwerte von Sensoren, die in Fig. 2 beschrieben sind, empfangen.

Die Ausgabe 506 kann eine Anzeige (Flüsssigkristallanzeige (Liquid Crystal Display, LCD), Kathodenstrahlröhrenanzeige (Cathode Ray Tube, CRT), Leuchtdiodenanzeige (Light Emitting Diode, LED) etc.; z. B. in einem Berührungsbildschirm enthalten), Lautsprecher etc. haben. Die Ausgabe kann weiterhin Kontrollsignale an Aktuatoren ausgeben, so wie dies in Fig. 2 beschrieben ist.

Der Speicher 507 kann eine Festplatte, einen Festkörperspeicher (Solid-State Drive, SSD), ein Flash-Laufwerk und dergleichen umfassen.

Die Kommunikationsschnittstelle 508 kann angepasst sein, um beispielsweise über ein Local Area Network (LAN), Wireless Local Area Network (WLAN), Mobiltelekommunikationssystem (GSM, UMTS, LTE, NR etc.), Bluetooth, Nahfeldkommunikation (Near-Field Communication, NFC), Infrarot etc. zu kommunizieren.

Es wird angemerkt, dass die obige Beschreibung nur eine beispielhafte Konfiguration des Computers 500 betrifft. Alternative Konfigurationen können mit zusätzlichen oder anderen Sensoren, Speichereinrichtungen, Schnittstellen oder dergleichen implementiert werden. Zum Beispiel kann die Kommunikationsschnittstelle 158 andere Funkzugangstechnologien als die erwähnten UMTS, LTE und NR unterstützen.

### BEZUGSZEICHENLISTE

- 1: Füllstandsbestimmungssystem
- 100: Steuergerät
- 101: Status-LED
- 102: Behälterbeleuchtung
- 103: Energiemanagementanordnung
- 104: Ladeeinrichtung
- 105: Ladekontroller
- 106: Batterie
- 107: Anzeige
- 108: Mikrocontroller
- 109: Kommunikationseinheit
- 110: WLAN
- 111: Mobilfunk
- 112: Sensorikanordnung
- 113: inertiale Messeinheit
- 114: NFC-Reader
- 115: Präsenzdetektor
- 116: Diagnosevorrichtung
- 200: Füllstandsmessanordnung
- 201: Elektronikeinheit (Elektronikkomponente)
- 201a: Messelektroden
- 201b: Umgebungsreferenzelektroden
- 201c: Fluidreferenzelektroden
- 202: Aktuator
- 203: standardisiertes Interface
- 204: Trägermaterial
- 205: Befestigungsmechanismus
- 206: Spannelement
- 206a: Federvorrichtung
- 208: Anschluss
- 209: Abdichtungsbereich
- 210: Einrastvorrichtung
- 211: Drehgelenk
- 212: Drehgelenk
- 300: Behälter
- 301: Schlauch
- 302: oberer Behälter
- 303: unterer Behälter
- 304: Übergang
- 306: RFID-Vorrichtung (NFC-Tag)
- 307: flexible Tasche
- 307b: Versteifung
- 307c: (rahmenförmige) Versteifung
- 308: steife Tasche
- 309: Gegenlager
- 310: Pfeile (Spannkraft)
- 400: Netzwerk
- 401: Server
- 402: Endgerät

## Patentansprüche

1. Behälter (300) für ein Füllstandsbestimmungssystem (1) zum Bestimmen eines Füllstandskriteriums eines medizinischen Fluides, wobei der Behälter (300) dazu eingerichtet ist, das medizinische Fluid aufzunehmen, der Behälter (300) umfassend
eine Halterung, mittels der eine Füllstandsmessanordnung (200) und der Behälter (300) miteinander verbindbar eingerichtet sind, wobei
die Halterung an dem Behälter (300) eine Tasche (307, 308) umfasst, die dazu ausgestaltet ist, die Füllstandsmessanordnung (200) nach einem Schwert-Scheide-Prinzip aufzunehmen.

2. Behälter (300) nach Anspruch 1, wobei die Tasche (307, 308) als flexible Tasche (307) ausgebildet ist.

3. Behälter (300) nach Anspruch 2, wobei der Behälter (300) ein Gegenlager (309, 210) umfasst, das dazu eingerichtet ist, eine an einer Füllstandsmessanordnung (200) vorgesehene rückenseitige Spannvorrichtung (206) in der Tasche (307, 308) zu fixieren.

4. Behälter (300) nach Anspruch 1, wobei die Tasche (307, 308) als versteifte Tasche (308) ausgebildet ist.

5. Behälter (300) nach einem der Ansprüche 1 bis 4, wobei der Behälter (300) als zumindest eines aus Urinbeutel, Urinmesssystem, Infusionsbeutel, Thoraxdrainage, Ventrikeldrainage oder chirurgische Drainage ausgebildet ist.

6. Behälter (300) nach einem der Ansprüche 1 bis 5, wobei der Behälter (300) eine RFID-Vorrichtung (306) umfasst.

7. Behälter (300) nach Anspruch 6, wobei die RFID-Vorrichtung (306) dazu eingerichtet ist, Informationen darüber zu speichern, ob der Behälter (300) mit einer Füllstandsmessanordnung (200) und einem Steuergerät (100) kompatibel ist und/oder ob der Behälter (300) bereits verwendet wurde.

8. Behälter (300) nach Anspruch 6 oder 7, wobei in der RFID-Vorrichtung (306) patientenbezogene Daten speicherbar sind.

9. Behälter (300) nach einem der Ansprüche 1 bis 8, wobei der Behälter (300) ein Auslassventil (304) umfasst, das dazu eingerichtet ist, durch einen an einer Füllstandsmessanordnung (200) vorgesehenen Aktuator (202) betätigt zu werden.

10. Behälter (300) für ein Füllstandsbestimmungssystem (1) zum Bestimmen eines Füllstandskriteriums eines medizinischen Fluides, wobei der Behälter (300) dazu eingerichtet ist, das medizinische Fluid aufzunehmen, und wobei der Behälter (300) so ausgebildet ist, dass
eine Füllstandsmessanordnung (200) mittels Adhäsion mit dem Behälter (300) verbindbar ist.

11. Behälter (300) für ein Füllstandsbestimmungssystem (1) zum Bestimmen eines Füllstandskriteriums eines medizinischen Fluides, wobei der Behälter (300) dazu eingerichtet ist, das medizinische Fluid aufzunehmen, und wobei
eine Füllstandsmessanordnung (200) auf den Behälter (300) aufgedruckt ist.

12. Füllstandsbestimmungssystem (1) zum Bestimmen eines Füllstandskriteriums eines medizinischen Fluides, umfassend
einen Behälter (300) nach einem der vorherigen Ansprüche, und
eine Füllstandsmessanordnung (200).
